# EUROPEAN PATENT SPECIFICATION

(11) **EP 2 103 600 B1**
(45) Date of publication and mention of the grant of the patent: **11.09.2013**
(21) Application number: 06842980.2
(22) Date of filing: 21.12.2006
(51) Int. Cl.: C07D 233/58, C07D 233/64, C07C 63/24, C07C 205/57, C08G 59/68

(54) **CLATHRATE COMPOUND, CURING CATALYST, COMPOSITION FOR FORMING CURED RESIN, AND CURED RESIN**
CLATHRATVERBINDUNG, KATALYSATOR ZUM HÄRTEN, ZUSAMMENSETZUNG ZUR BILDUNG EINES GEHÄRTETEN HARZES UND GEHÄRTETES HARZ
COMPOSÉ DE CLATHRATE, CATALYSEUR DURCISSEUR, COMPOSITION DE FORMATION DE RÉSINES DURCIES, ET RÉSINES DURCIES

(43) Date of publication of application: 23.09.2009
(73) Proprietor: Nippon Soda Co., Ltd., Tokyo 100-8165 (JP)
(72) Inventor: KANEKO, Masami, Ichihara-shi Chiba 2900045 (JP); AMANOKURA, Natsuki, Ichihara-shi Chiba 290-0045 (JP)
(74) Representative: Wibbelmann, Jobst
(86) International application number: PCT/JP2006/325472
(87) International publication number: WO 2008/075427

(56) References cited:
- JP-A- 08 151 372
- JP-A- 10 316 639
- JP-A- 10 511 718
- JP-A- 56 100 748
- JP-A- 57 135 870
- JP-A- 58 076 420
- JP-A- 60 252 620
- JP-A- 2004 300 041
- JP-A- 2004 503 632
- JP-A- 2007 039 449
- US-A- 3 746 686
- CHEN, ZHANG, FENG, FU: "Synthesis, structures of cobalt/copper complexes and magnetic property of copper complex with the mixed ligands 5-nitro-1,3-benzenedicarboxylic acid and imidazole", INORGANIC CHEMISTRY COMMUNICATIONS, vol. 9, 18 January 2006 (2006-01-18), pages 300-303, XP002631183,

## Description

### TECHNICAL FIELD

The present invention relates to a novel clathrate compound, a curing catalyst containing the clathrate compound, a composition for forming a cured resin that uses the curing catalyst, a method of producing a cured resin that uses the composition for forming a cured resin, and a cured resin obtained using the production method.

### BACKGROUND ART

Epoxy resins have excellent mechanical properties and thermal properties, and are therefore widely used in all manner of fields. An imidazole is typically used as the curing catalyst for curing these epoxy resins, but in epoxy resin-imidazole mixed liquids, curing initiation tends to be very fast, which creates a problem in that the one-pot stability is extremely poor.

Accordingly, as an alternative curing agent, the use of an acid addition salt of an imidazole obtained by adding a hydroxybenzoic acid to an imidazole (see Patent Document 1), the use of a clathrate of a tetrakisphenol compound (such as 1,1,2,2-tetrakis(4-hydroxyphenyl)ethane (hereafter abbreviated as "TEP")) and an imidazole (see Patent Document 2), and the use of isophthalic acid imidazole clathrate (see Patent Document 3) have been proposed. These acid addition salts of an imidazole and clathrates do provide a certain amount of effect, but the development of additional catalysts having either similar functionality or superior functionality has been keenly sought.

Patent Document 1:
   Japanese Examined Patent Application, Second Publication No. Hei 04-2638
Patent Document 2:
   Japanese Unexamined Patent Application, First Publication No. Hei 11-71449
Patent Document 3:
   US 3,746,686

### DISCLOSURE OF INVENTION

### PROBLEMS TO BE SOLVED BY THE INVENTION

An object of the present invention is to provide a curing catalyst (a clathrate compound) for which the curing reaction can be suppressed at low temperatures, allowing an improvement in the one-pot stability, but which can effectively cure a resin upon heat treatment. Furthermore, the present invention also provides a composition for forming a cured resin that uses the above curing catalyst, a method of producing a cured resin that uses the composition for forming a cured resin, and a cured resin obtained using the production method.

### MEANS TO SOLVE THE PROBLEMS

As a result of intensive research aimed at achieving the above objects, the inventors of the present invention discovered that the above objects could be achieved by using a clathrate compound containing at least a specific imidazole and a specific acid, and the inventors were therefore able to complete the present invention.

In other words, the present invention relates to:
(1) a clathrate compound containing at least an isophthalic acid compound represented by formula (I) shown below:

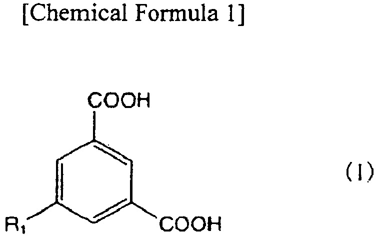

[wherein R₁ represents a C1 to C6 alkyl group, C1 to C6 alkoxy group, nitro group or hydroxyl group], and an imidazole compound represented by formula (II) shown below:

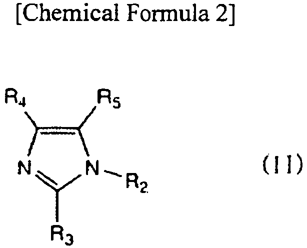

[wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent]; provided that the clathrate compound is neither [Co(5-nbdc)₂(imH)₂]ₙ nor [Cu₃(5-nbdc)₂(OH)₂(imH)₂]ₙ, wherein 5-nbdc H₂ represents 5-nitro-1,3-benzene dicarboxylic acid and imH represents imidazole;
(2) the compound disclosed in (1) above, wherein the isophthalic acid compound represented by formula (I) is 5-t-butylisophthalic acid or 5-nitroisophthalic acid;
(3) the compound disclosed in (1) or (2) above, wherein the imidazole compound represented by formula (II) is 2-ethyl-4-methylimidazole, 2-methylimidazole, 1-benzyl-2-methylimidazole, 2-heptadecylimidazole, 2-undecylimidazole or 2-phenyl-4-methyl-5-hydroxymethylimidazole;
(4) the compound disclosed in any one of (1) to (3) above, wherein R₂ in formula (II) is a hydrogen atom;
(5) the compound disclosed in any one of (1) to (4) above, wherein the compound is in a powdered form; and
(6) a curing catalyst for an epoxy resin, containing a clathrate compound disclosed in any one of (1) to (5) above.

Furthermore, the present invention also relates to:
(7) a composition for forming a cured epoxy resin, containing:
   (A) an epoxy resin, and
   (B) a clathrate compound containing at least an isophthalic acid compound represented by formula (I) shown below:

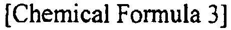

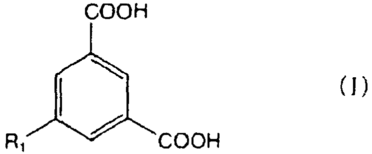

[wherein R₁ represents a C1 to C6 alkyl group, C1 to C6 alkoxy group, nitro group or hydroxyl group], and an imidazole compound represented by formula (II) shown below:

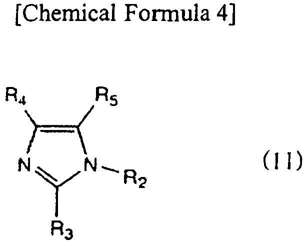

[wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent];
(8) the composition for forming a cured epoxy resin disclosed in (7) above, wherein an amount of the imidazole compound represented by formula (II) within component (B) is within a range from 0.01 to 1.0 mol relative to 1 mol of epoxy rings within the epoxy resin of component (A);
(9) the composition for forming a cured epoxy resin disclosed in (7) or (8) above, wherein the isophthalic acid compound represented by formula (I) is 5-t-butylisophthalic acid or 5-nitroisophthalic acid; and
(10) the composition for forming a cured epoxy resin disclosed in any one of (7) to (9) above, wherein the imidazole compound represented by formula (II) is 2-ethyl-4-methylimidazole, 2-methylimidazole, 1-benzyl-2-methylimidazole, 2-heptadecylimidazole, 2-undecylimidazole or 2-phenyl-4-methyl-5-hydroxymethylimidazole.

Moreover, the present invention also relates to:
(11) a method of producing a cured epoxy resin, including curing a composition for forming a cured epoxy resin disclosed in any one of (7) to (10) above by conducting a heat treatment;
(12) the method of producing a cured epoxy resin disclosed in (11) above, wherein a heating temperature during the heat treatment is within a range from 60 to 250°C; and
(13) a cured epoxy resin obtained using a method disclosed in (11) or (12) above.

In addition, the present invention also relates to:
(14) a compound that can be obtained after dissolving or suspending at least an isophthalic acid compound represented by formula (I) shown below:

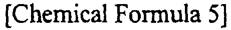

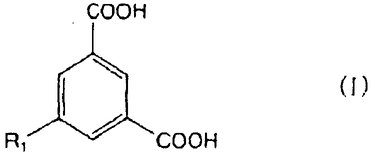

[wherein R₁ represents a C 1 to C6 alkyl group, C1 to C6 alkoxy group, nitro group or hydroxyl group], and an imidazole compound represented by formula (II) shown below:

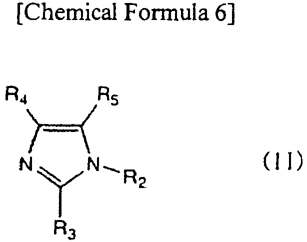

[wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C 1 to C20 alkyl group, phenyl group, benzyl group or C 1 to C20 acyl group that may have a substituent] in a solvent and subsequently conducting heating, provided that the clathrate compound is neither [Co(5-nbdc)₂ (imH)₂]ₙ nor [Cu₃(5-nbcd)₂(OH)₂ (imH)₂]ₙ wherein 5-nbcdH₂ represents 5-nitro-1,3-benzenedicarboxylic acid and imH represents imidazole;
(15) the compound disclosed in (14) above, that can be obtained by dissolving or suspending an isophthalic acid compound represented by formula (I) and an imidazole compound represented by formula (II) in a solvent, conducting heating, and then performing a crystallization;
(16) use of a host compound for a clathrate compound, characterized in that the host compound is represented by formula (I) shown below:

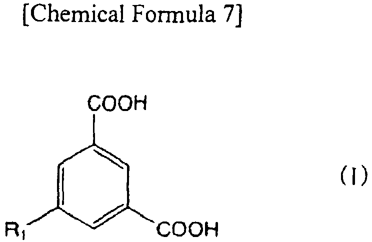

[wherein R₁ represents a nitro group or a C4 alkyl group] provided that the clathrate compound is neither [Co(5-nbcd)₂(imH)₂]ₙ nor [Cu₃(5-nbdc)₂(OH)₂(imH)₂]ₙ, wherein 5-nbdc H₂ represents 5-nitro-1,3-benzenedicarboxylic acid and imH represents imidazole; and
(17) the use disclosed in (16) above, wherein the C4 alkyl group is a t-butyl group;
(18) use of a clathrate compound comprising at least an isophthalic acid compound represented by a formula (I) shown below:
   wherein R₁ represents a C1 to C6 alkyl group, C1 to C6 alkoxy group, nitro group or hydroxyl group, and an imidazole compound represented by a formula (II) shown below:
   wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent, as curing catalyst.

### EFFECTS OF THE INVENTION

According to a curing catalyst (clathrate compound) of the present invention, the curing reaction can be suppressed at low temperatures, allowing an improvement in the one-pot stability, whereas a resin can be cured effectively by conducting a heat treatment.

### BRIEF DESCRIPTION OF THE DRAWINGS

FIG. 1 is a thermal analysis (TG/DTA) chart for a clathrate according to an example 1 of the present invention.
FIG. 2 is a thermal analysis (DSC) chart upon temperature variation for the clathrate according to example 1 of the present invention.
FIG. 3 is a thermal analysis (DSC) chart at a fixed temperature (80°C) for the clathrate according to example 1 of the present invention.
FIG. 4 is a thermal analysis (TG/DTA) chart for a clathrate according to an example 2 of the present invention.
FIG. 5 is a thermal analysis (DSC) chart upon temperature variation for the clathrate according to example 2 of the present invention.
FIG. 6 is a thermal analysis (DSC) chart at a fixed temperature (80°C) for the clathrate according to example 2 of the present invention.
FIG. 7 is a thermal analysis (TG/DTA) chart for a clathrate according to an example 4 of the present invention.
FIG. 8 is a thermal analysis (DSC) chart upon temperature variation for the clathrate according to example 4 of the present invention.
FIG. 9 is a thermal analysis (DSC) chart at a fixed temperature (80°C) for the clathrate according to example 4 of the present invention.
FIG. 10 is a thermal analysis (TG/DTA) chart for only 2-undecylimidazole.
FIG. 11 is a thermol analysis (TG/DTA) chart for a clathrate according to an example 5 of the present invention.
FIG. 12 is a thermal analysis (DSC) chart upon temperature variation for 2-undecylimidazole and an epoxy resin.
FIG. 13 is a thermal analysis (DSC) chart upon temperature variation for the clathrate according to an example 5 of the present invention and an epoxy resin.
FIG. 14 is a thermal analysis (TG/DTA) chart for only 2-heptadecylimidazole.
FIG. 15 is a thermal analysis (TG/DTA) chart for a clathrate according to an example 6 of the present invention.
FIG. 16 is a thermal analysis (DSC) chart upon temperature variation for 2-heptadecylimidazole and an epoxy resin.
FIG. 17 is a thermal analysis (DSC) chart upon temperature variation for the clathrate according to an example 6 of the present invention and an epoxy resin.
FIG. 18 is a ¹H-NMR spectral chart for the clathrate according to example 1 of the present invention.
FIG. 19 illustrates X-ray diffraction patterns for the clathrate (5-NO2IPA-2E4MZ) according to example 1 of the present invention and 5-nitroisophthalic acid (5-NO2-IPA).

### BEST MODE FOR CARRYING OUT THE INVENTION

There are no particular restrictions on the clathrate compound of the present invention, provided it includes at least an isophthalic acid compound represented by formula (I) and an imidazole compound represented by formula (II). The compound may also include a third component such as a solvent, although the quantity of this third component is preferably not more than 40 mol%, more preferably 35 mol% or less, still more preferably 20 mol% or less, and still more preferably 10 mol% or less. A clathrate compound that does not include a third component and is composed solely of the isophthalic acid compound represented by formula (I) and the imidazole compound represented by formula (II) is the most desirable. In the present invention, a "clathrate compound" describes a compound in which two, or three or more, different types of molecule are bonded together via bonds other than covalent bonds, and preferably describes a crystalline compound in which two, or three or more, different types of molecule are bonded together via bonds other than covalent bonds. A clathrate compound of the present invention containing an isophthalic acid compound represented by formula (I) and an imidazole compound represented by formula (II) can also be described as a salt formed from the isophthalic acid compound of formula (I) and the imidazole compound represented by formula (II).
The clathrate compound of the present invention can be used as a resin curing agent for polyester resins, epoxy resins and epoxy-polyester resins and the like, and is particularly ideal as a curing agent for epoxy resins. Furthermore, the clathrate compound of the present invention may be in a liquid form prepared by dissolving the compound in a solvent, but is preferably in a powdered form (precipitated from within a solvent). If the compound is in a powdered form, then it may also be used in powdered paints and the like.

A description of the isophthalic acid compound represented by formula (I) is presented below. In formula (I), R₁ represents a C1 to C6 alkyl group, C1 to C6 alkoxy group, nitro group or hydroxyl group.

The C1 to C6 alkyl group is preferably a C1 to C4 alkyl group, and may have a substituent. Specific examples of the C1 to C6 alkyl group include a methyl group, ethyl group, propyl group, isopropyl group, cyclopropyl group, butyl group, isobutyl group, s-butyl group, t-butyl group, cyclobutyl group, cyclopropylmethyl group, pentyl group, isopentyl group, 2-methylbutyl group, neopentyl group, 1-ethylpropyl group, hexyl group, isohexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2,3-dimethylbutyl group, 1-ethylbutyl group and 2-ethylbutyl group.

The C1 to C6 alkoxy group is preferably a C1 to C4 alkoxy group, and may have a substituent. Specific examples of the C1 to C6 alkoxy group include a methoxy group, ethoxy group, propoxy group, isopropoxy group, butoxy group, isobutoxy group, s-butoxy group, t-butoxy group, pentoxy group, isopentoxy group, 2-methylbutoxy group, 1-ethylpropoxy group, 2-ethylpropoxy group, neopentoxy group, hexyloxy group, 4-methylpentoxy group, 3-methylpentoxy group, 2-methylpentoxy group, 3,3-dimethylbutoxy group, 2,2-dimethylbutoxy group, 1,1-dimethylbutoxy group, 1,2-dimethylbutoxy group, 1,3-dimethylbutoxy group and 2,3-dimethylbutoxy group.

Specific examples of preferred compounds for the isophthalic acid compound represented by formula (1) include 5-t-butylisophthalic acid and 5-nitroisophthalic acid.

Next is a description of the imidazole compound represented by formula (II). In formula (II), R₂ represents a hydrogen atom, C1 to C10 alkyl group, phenyl group, benzyl group or cyanoethyl group, and of these, a hydrogen atom is preferred.

The C1 to C 10 alkyl group is preferably a C1 to C6 alkyl group, and may have a substituent. Specific examples of the C1 to C 10 alkyl group include the alkyl groups listed above, as well as a heptyl group, octyl group, nonyl group and decyl group.
Further, the phenyl group and benzyl group may also have a substituent.

R₃ to R₅ each independently represents a hydrogen atom, nitro group, halogen atom, or a C1 to C20 alkyl group, phenyl group, benzyl group or C1 to C20 acyl group that may have a substituent, preferably each independently represents a hydrogen atom, nitro group, halogen atom, or a C 1 to C 17 alkyl group, phenyl group, benzyl group or C1 to C 17 acyl group that may have a substituent, and more preferably each independently represents a hydrogen atom, nitro group, halogen atom, or a C1 to C10 alkyl group, phenyl group, benzyl group or C 1 to C10 acyl group that may have a substituent. The C1 to C20 alkyl group is as described above. The C1 to C20 acyl group that may have a substituent is preferably a C1 to C10 acyl group that may have a substituent, and is more preferably a C1 to C6 acyl group that may have a substituent. Specific examples include a formyl group, acetyl group, propionyl group, butyryl group, valeryl group or benzoyl group.
There are no particular restrictions on the substituent that may be bonded to the alkyl group, phenyl group, benzyl group or acyl group, provided that a solid compound can be obtained that contains at least the isophthalic acid compound represented by formula (I) and the imidazole compound represented by formula (II) as structural elements. One example of a preferred substituent is a hydroxyl group.

Specific examples of the imidazole compound represented by formula (II) include 2-ethyl-4-methylimidazole, 2-methylimidazole, 1-benzyl-2-methylimidazole, 2-heptadecylimidazole, 2-undecylimidazole, 2-phenyl-4-methyl-5-hydroxymethylimidazole, 2-phenylimidazole, 2-phenyl-4-methylimidazole, 1-benzyl-2-phenylimidazole, 1,2-dimethylimidazole, 1-cyanoethyl-2-methylimidazole, 1-cyanoethyl-2-ethyl-4-methylimidazole, 1-cyanoethyl-2-undecylimidazole, 1-cyanoethyl-2-phenylimidazole and 2-phenyl-4,5-dihydroxymethylimidazole. In terms of the ease with which a powdered clathrate compound can be formed, 2-ethyl-4-methylimidazole and 2-methylimidazole are preferred, and if the one-pot stability is also taken into consideration, then 2-ethyl-4-methylimidazole is particularly desirable.

The above type of clathrate compound of the present invention can be obtained by adding the isophthalic acid compound represented by formula (I) and the imidazole compound represented by formula (II) to a solvent, and then conducting either a heat treatment or a heated reflux treatment, under stirring if required, to precipitate the clathrate compound. Furthermore, depending on the variety of isophthalic acid compound represented by formula (I) and the variety of the imidazole compound represented by formula (II), precipitation via the same operation as that described above may yield a crystalline compound.
In order to facilitate dissolution within the solvent, the isophthalic acid compound represented by formula (I) and the imidazole compound represented by formula (II) are preferably dissolved separately in solvents, and the resulting solutions are then preferably mixed. Examples of solvents that may be used include water, methanol, ethanol, ethyl acetate, methyl acetate, diethyl ether, dimethyl ether, acetone, methyl ethyl ketone and acetonitrile. In terms of the proportions added of the isophthalic acid compound represented by formula (I) and the imidazole compound represented by formula (II) during production of the clathrate compound of the present invention, the amount added of the imidazole compound represented by formula (II) (the guest) is preferably within a range from 0.1 to 5.0 mol, and more preferably from 0.5 to 3.0 mol, relative to 1 mol of the isophthalic acid compound represented by formula (I) (the host).

There are no particular restrictions on the compound of the present invention, provided it can be obtained after dissolving or suspending at least the isophthalic acid compound represented by formula (I) and the imidazole compound represented by formula (II) in a solvent and conducting heating. The compound may also include a third component such as a solvent, although the quantity of this third component is preferably not more than 40 mol%, more preferably 35 mol% or less, still more preferably 20 mol% or less, and still more preferably 10 mol% or less, and a compound that does not contain a third component is the most desirable.

Although there are no particular restrictions on the compound of the present invention, provided it can be obtained after dissolving or suspending at least the isophthalic acid compound represented by formula (I) and the imidazole compound represented by formula (II) in a solvent and conducting heating, the compound of the present invention is preferably a compound that can be obtained by dissolving or suspending at least the isophthalic acid compound represented by formula (I) and the imidazole compound represented by formula (II) in a solvent, conducting heating, and then precipitating the compound, and is more preferably a crystalline compound that can be obtained by dissolving or suspending at least the isophthalic acid compound represented by formula (I) and the imidazole compound represented by formula (II) in a solvent, conducting heating, and then crystallizing the compound.

The isophthalic acid compound represented by formula (I) and the imidazole compound represented by formula (II) are as described above. There are no particular restrictions on the solvent, provided it does not hinder the process of obtaining the compound of the present invention by dissolving or suspending the isophthalic acid compound represented by formula (I) and the imidazole compound represented by formula (II) in a solvent and conducting heating, and an appropriate solvent can be selected in accordance with the isophthalic acid compound represented by formula (I) and the imidazole compound represented by formula (II) that are actually used. Specific examples of the solvent are as described above.

In terms of the proportions added of the isophthalic acid compound represented by formula (I) and the imidazole compound represented by formula (II) during production of the compound of the present invention, the amount added of the imidazole compound represented by formula (II) is preferably within a range from 0.1 to 5.0 mol, and more preferably from 0.5 to 3.0 mol, relative to 1 mol of the isophthalic acid compound represented by formula (I).

During production of the compound of the present invention, the isophthalic acid compound represented by formula (I) and the imidazole compound represented by formula (II) are dissolved or suspended in a solvent, and both compounds are preferably dissolved in the solvent. In those cases where both compounds are dissolved in a solvent, the entire amount of both compounds need not necessarily dissolve in the solvent, but at least a small portion of both compounds must dissolve in the solvent.

There are no particular restrictions on the heating conditions employed during production of the compound of the present invention, provided that the compound of the present invention can be obtained after dissolving at least the isophthalic acid compound represented by formula (I) and the imidazole compound represented by formula (II) in a solvent and conducting the heating. For example, heating may be conducted at a temperature within a range from 40 to 120°C, and is preferably conducted within a range from 50 to 90°C.
Furthermore, the heating conducted during production of the compound of the present invention need not necessarily be conducted while stirring the solution or suspension containing the isophthalic acid compound represented by formula (I) and the imidazole compound represented by formula (II), but the heating is preferably conducted while the solution or suspension is stirred, and is more preferably conducted under heated reflux conditions.

During the production of the compound of the present invention, there are no particular restrictions on the step conducted after dissolving or suspending at least the isophthalic acid compound represented by formula (I) and the imidazole compound represented by formula (II) in a solvent and conducting heating, provided this subsequent step yields a solid compound containing at least the isophthalic acid compound represented by formula (I) and the imidazole compound represented by formula (II) as structural elements. For example, after dissolving the isophthalic acid compound represented by formula (I) and the imidazole compound represented by formula (II) in a solvent and conducting heating, the solid compound may be precipitated by simply stopping the heating treatment, but the solution is preferably left to stand over night at room temperature after the heating is stopped. After precipitation of the solid compound, filtering and drying can be used to obtain the target compound. Furthermore, depending on factors such as the types of the isophthalic acid compound represented by formula (I) used and the types of the imidazole compound represented by formula (II) used, the same operations as those described in the above steps for obtaining the solid compound may yield a crystalline compound of the present invention.
Provided a compound is the same as the compound of the present invention, it is deemed to be incorporated within the present invention, even if it is not obtained after dissolving at least an isophthalic acid compound represented by formula (I) and an imidazole compound represented by formula (II) in a solvent and conducting heating.

There are no particular restrictions on the curing catalyst for an epoxy resin according to the present invention, provided it includes a clathrate compound of the present invention or a compound of the present invention, and for example, the catalyst may also include other epoxy resin curing catalysts.

Furthermore, there are no particular restrictions on the composition for forming a cured epoxy resin according to the present invention, provided the composition includes an epoxy resin (component (A)) and either a clathrate compound of the present invention or a compound of the present invention (component (B)). The component (B) is as described above.

As the epoxy resin of component (A), all manner of conventional polyepoxy compounds can be used, and specific examples include aromatic glycidyl ether compounds such as bis(4-hydroxyphenyl)propane diglycidyl ether, bis(4-hydroxy-3,5-dibromophenyl)propane diglycidyl ether, bis(4-hydroxyphenyl)ethane diglycidyl ether, bis(4-hydroxyphenyl)methane diglycidyl ether, resorcinol diglycidyl ether, phloroglucinol triglycidyl ether, trihydroxybiphenyl triglycidyl ether, tetraglycidylbenzophenone, bisresorcinol tetraglycidyl ether, tetramethylbisphenol A diglycidyl ether, bisphenol C diglycidyl ether, bisphenolhexafluoropropane diglycidyl ether, 1,3-bis[1-(2,3-epoxypropoxy)-1-trifluoromethyl-2,2,2-trifluoroethyl]benzene, 1,4-bis[1-(2,3-epoxypropoxy)-1-trifluoromethyl-2,2,2-trifluoromethyl]benzene, 4,4'-bis(2,3-epoxypropoxy)octafluorobiphenyl, and phenol novolak bisepoxy compounds; alicyclic polyepoxy compounds such as alicyclic diepoxy acetal, alicyclic diepoxy adipate, alicyclic diepoxy carboxylate, and vinylcyclohexene dioxide; glycidyl ester compounds such as diglycidyl phthalate, diglycidyl tetrahydrophthalate, diglycidyl hexahydrophthalate, dimethylglycidyl phthalate, dimethylglycidyl hexahydrophthalate, diglycidyl p-oxybenzoate, diglycidyl cyclopentane-1,3-dicarboxylate, and dimer acid glycidyl ester; glycidylamine compounds such as diglycidylaniline, diglycidyltoluidine, triglycidylaminophenol, tetraglycidyldiaminodiphenylmethane, and diglycidyltribromoanaline; and heterocyclic epoxy compounds such as diglycidylhydantoin, glycidylglycidoxyalkylhydantoin, and triglycidyl isocyanurate.

The proportion of the imidazole compound represented by formula (II) within the components (A) and (B) in the composition for forming a cured epoxy resin according to the present invention is preferably such that the amount of the imidazole compound represented by formula (II) within the component (B) is within a range from 0.01 to 1.0 mol, more preferably from 0.1 to 1.0 mol, and still more preferably from 0.3 to 1.0 mol, relative to 1 mol of epoxy rings within the epoxy resin of the component (A).

Further, the composition for forming a cured epoxy resin according to the present invention can be produced by mixing the component (A) and the component (B), and in order to ensure formation of a satisfactory mixed state, mixing is usually conducted under heating at a temperature of 60 to 100°C. In the production of the cured epoxy resin, the one-pot stability of the composition at this temperature is an important factor.

Furthermore, there are no particular restrictions on the method used for producing the cured epoxy resin of the present invention, provided the method includes curing the composition for forming a cured epoxy resin by conducting a heat treatment. The heating temperature employed during the heat treatment is typically within a range from 60 to 250°C and preferably from 100 to 200°C, and the composition is preferably cured in a short period of time at such a temperature.

There are no particular restrictions on the host compound for the clathrate compound of the present invention, provided it is an isophthalic acid compound represented by formula (I) (wherein R₁ represents a nitro group or a C4 alkyl group), and the C4 alkyl group is preferably a t-butyl group.
In the present invention, the host compound for the clathrate compound refers to a compound that undergoes bonding other than covalent bonding to one, or two or more, different types of molecules (such as a guest or solvent molecule) to form a compound, wherein this compound is capable of forming a clathrate lattice, and more preferably refers to a compound that undergoes bonding other than covalent bonding to one, or two or more, different types of molecules (such as a guest or solvent molecule) to form a crystalline compound, wherein this crystalline compound is capable of forming a clathrate lattice. Here, a "clathrate lattice" refers to either a structure in which molecules of the host compound are bonded together via bonding other than covalent bonding, and another molecule (such as a guest or solvent molecule) or a combination of another molecule and a host compound are bonded by some form of bonding other than covalent bonding within the spaces between two, or three or more, host compounds, or a structure in which the host compound is bonded to another molecule (such as a guest or solvent molecule) via bonding other than covalent bonding, and a host compound and/or another molecule (such as a guest or solvent molecule) are bonded by some form of bonding other than covalent bonding within the spaces between two, or three or more, of the host compounds bonded to other molecules. When preparing a clathrate compound using a host compound of the present invention, depending on the types of the guest compound, molecules of the guest compound may also bond together via some form of bonding other than covalent bonding, but such bonding has no effect on the host compound of the present invention acting as the host compound.
There are no particular restrictions on the shape of the clathrate lattice, and examples include tunnel-type lattices, layered lattices and network lattices.
The host compound of the present invention forms a lattice structure within at least a portion of the clathrate compound, and host compound molecules that do not form a clathrate lattice may be included within the clathrate compound, although the entire clathrate compound is preferably in the form of a clathrate lattice.

### EXAMPLES

A more detailed description of the present invention is presented below based on a series of examples, although the technical scope of the present invention is in no way limited by these examples.

### [Example 1]

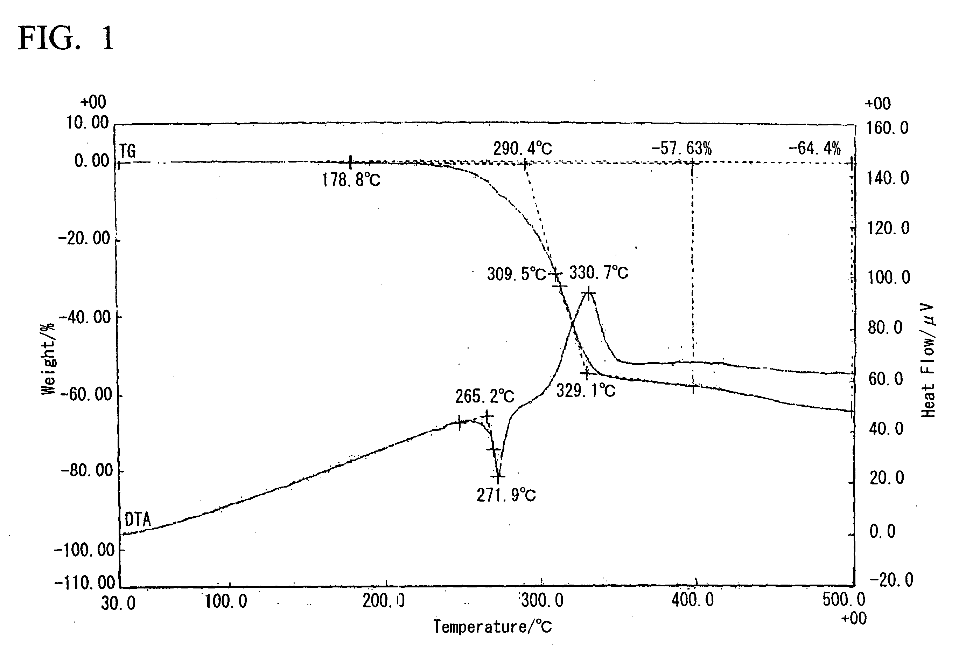

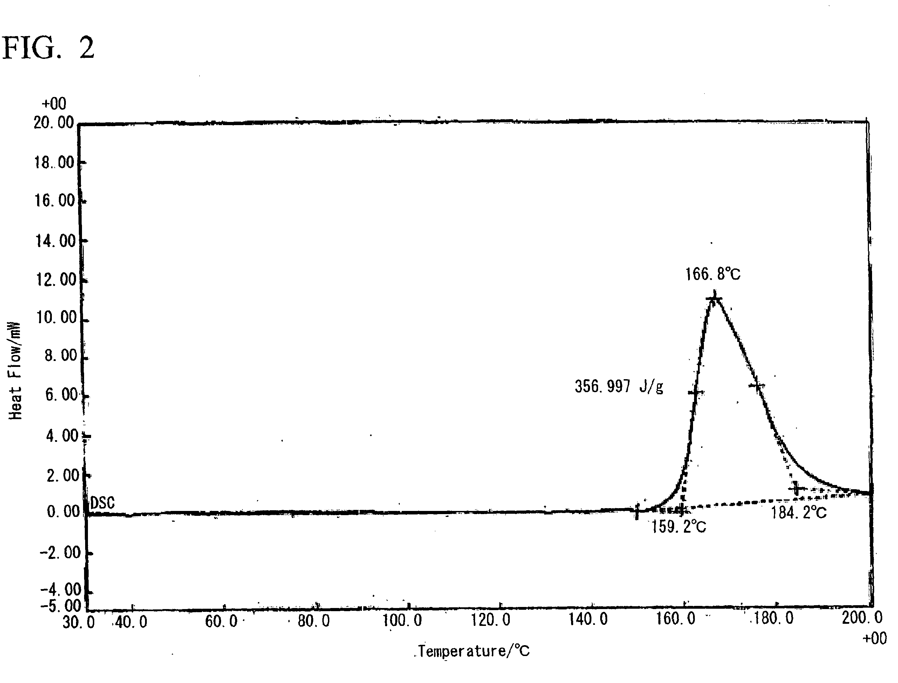

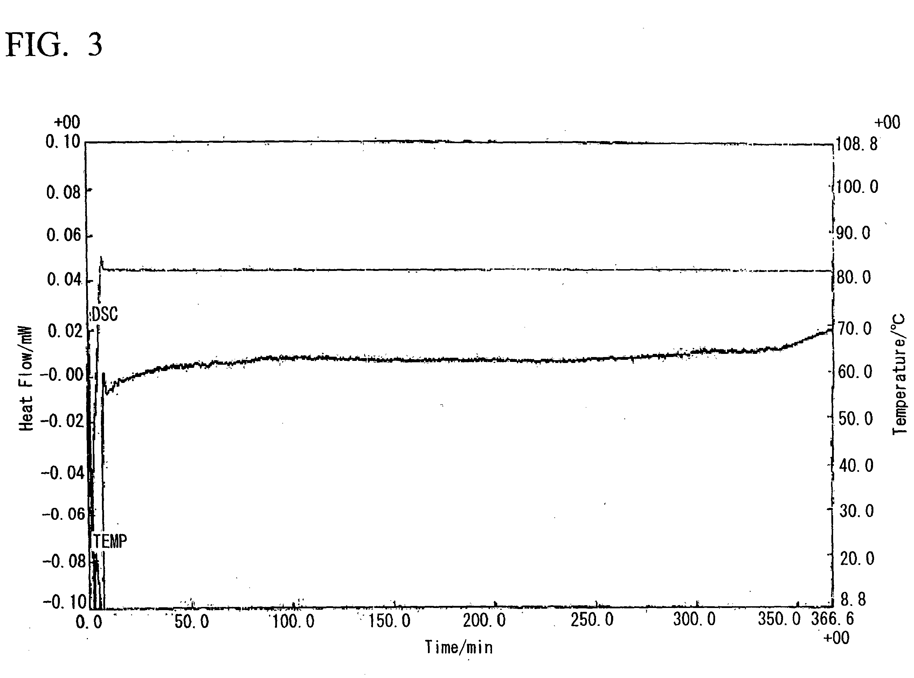

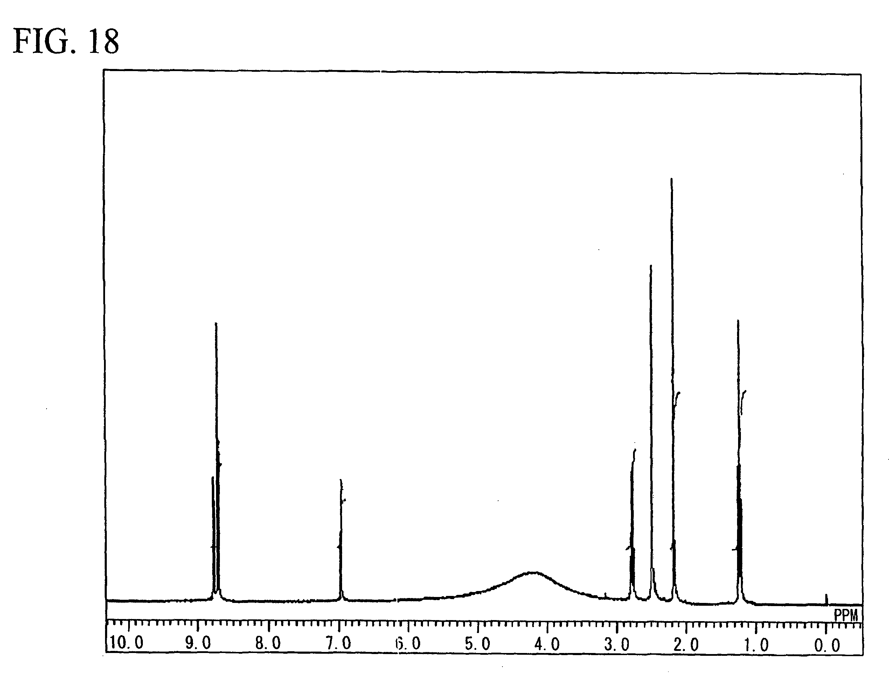

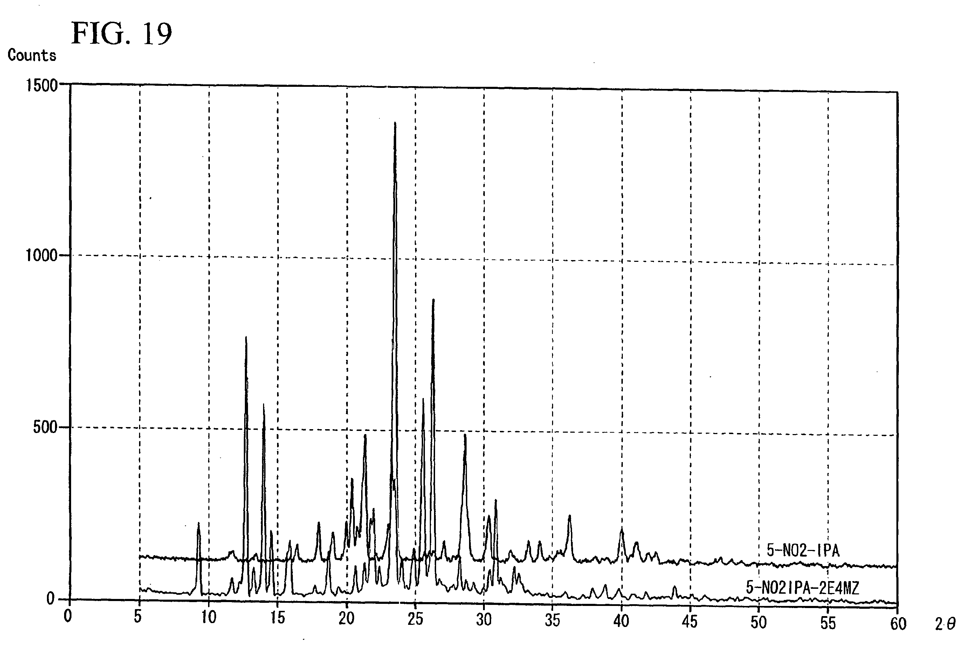

20 ml of a methanol solution containing 10 mmol (1.10 g) of 2-ethyl-4-methylimidazole was added to 20 ml of a methanol solution containing 5 mmol (1.05 g) of 5-nitroisophthalic acid under conditions of heated reflux with stirring. Although heating is subsequently stopped, crystals precipitate almost immediately, the mixture was left to stand overnight at room temperature, and then filtered and dried under vacuum, yielding a clathrate (0.5 g, 33%). Analysis of the obtained clathrate by NMR revealed 1:1 clathrate crystals. The ¹H-NMR spectral chart and the X-ray diffraction pattern for the obtained clathrate (5-NO2IPA-2E4MZ) are shown in FIG. 18 and FIG. 19 respectively. For the purposes of comparison, the X-ray diffraction pattern for 5-nitroisophthalic acid (5-NO2-IPA) is also shown in FIG. 19. A thermal analysis (TG/DTA) chart for the obtained clathrate crystals is shown in FIG. 1. Furthermore, a thermal analysis (DSC) chart upon temperature variation for the obtained clathrate crystals is shown in FIG. 2, whereas a thermal analysis (DSC) chart at a fixed temperature (80°C) is shown in FIG. 3.

### [Example 2]

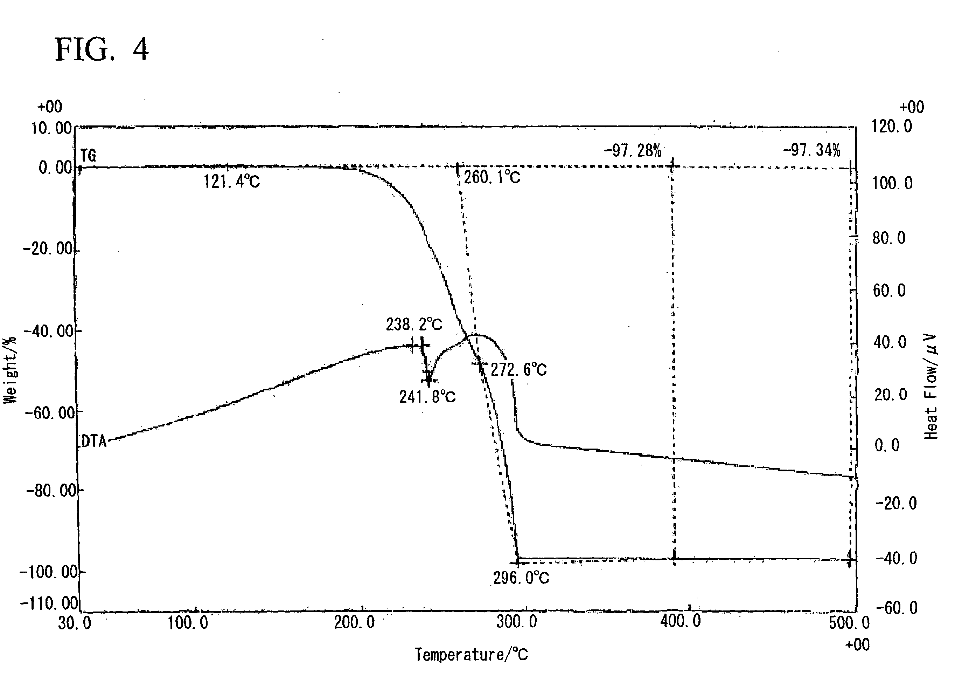

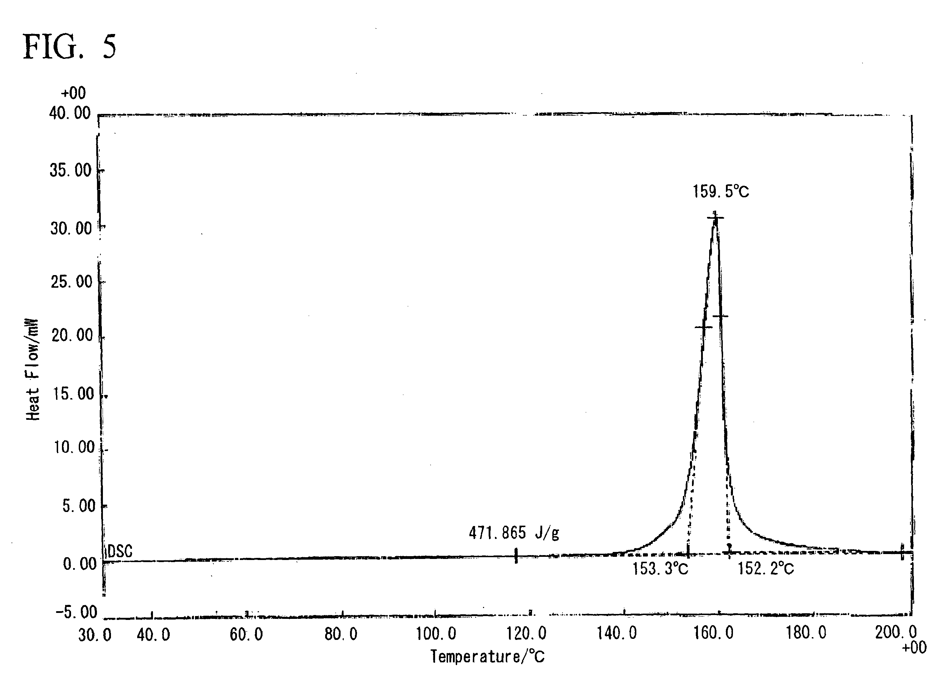

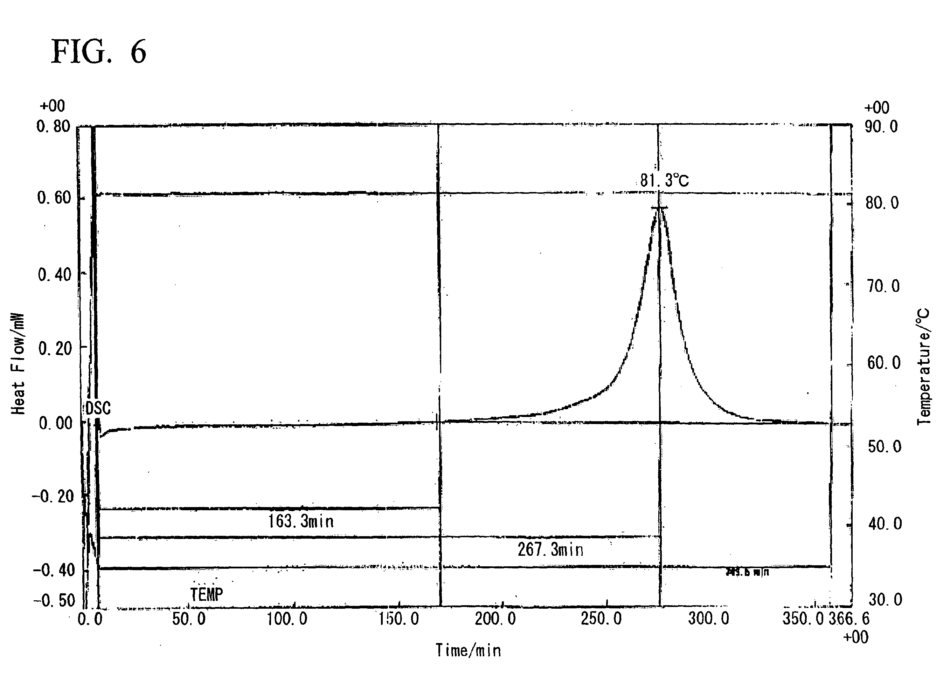

15 mmol (3.33 g) of 5-t-butylisophthalic acid and 18 mmol (1.98 g, 1.2 eq.) of 2-ethyl-4-methylimidazole were added to 60 ml of methanol, and the resulting mixture was stirred under heated reflux in a round-bottom flask for 30 minutes, thereby dissolving the crystals. Subsequently, the solution was left to stand at room temperature, and the crystals that precipitated from the solution were filtered and dried under vacuum, yielding a clathrate compound (2.34 g, 47%). Analysis of the obtained clathrate by NMR revealed 1:1 clathrate crystals. A thermal analysis (TG/DTA) chart for the obtained clathrate crystals is shown in FIG. 4. Furthermore, a thermal analysis (DSC) chart upon temperature variation for the obtained clathrate crystals is shown in FIG. 5, whereas a thermal analysis (DSC) chart at a fixed temperature (80°C) is shown in FIG. 6.

### [Example 3]

With the exception of altering the amount of 2-ethyl-4-methylimidazole to 16.5 mmol (1.81 g, 1.1 eq.), a clathrate was prepared in the same manner as example 2 (2.08 g, 42%). Analysis of the obtained clathrate by NMR revealed 1:1 clathrate crystals, and a thermal analysis (TG/DTA) chart for the obtained clathrate crystals was the same as that for the crystals obtained in example 2.

### [Example 4]

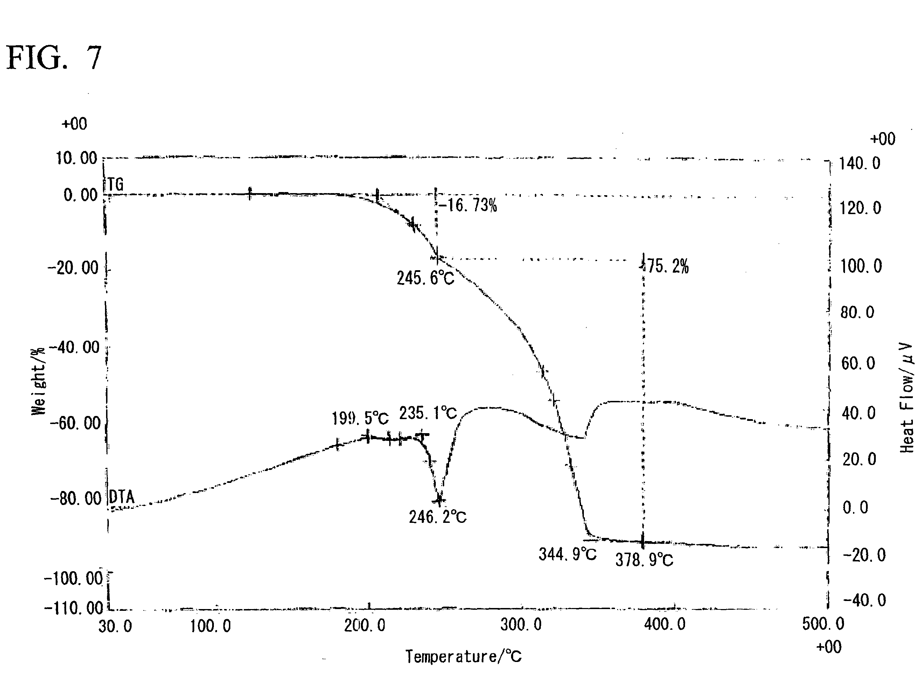

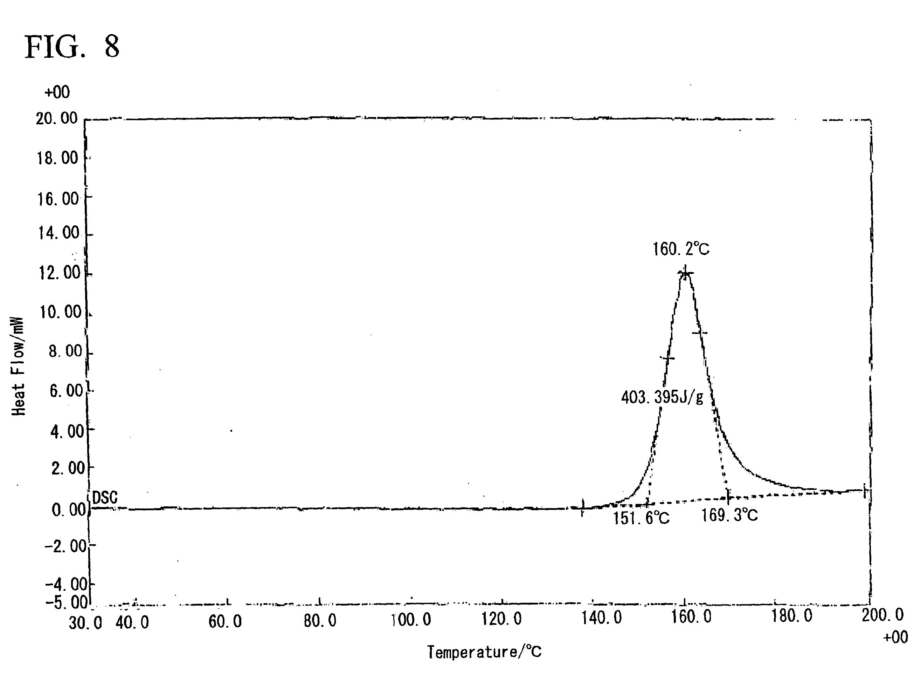

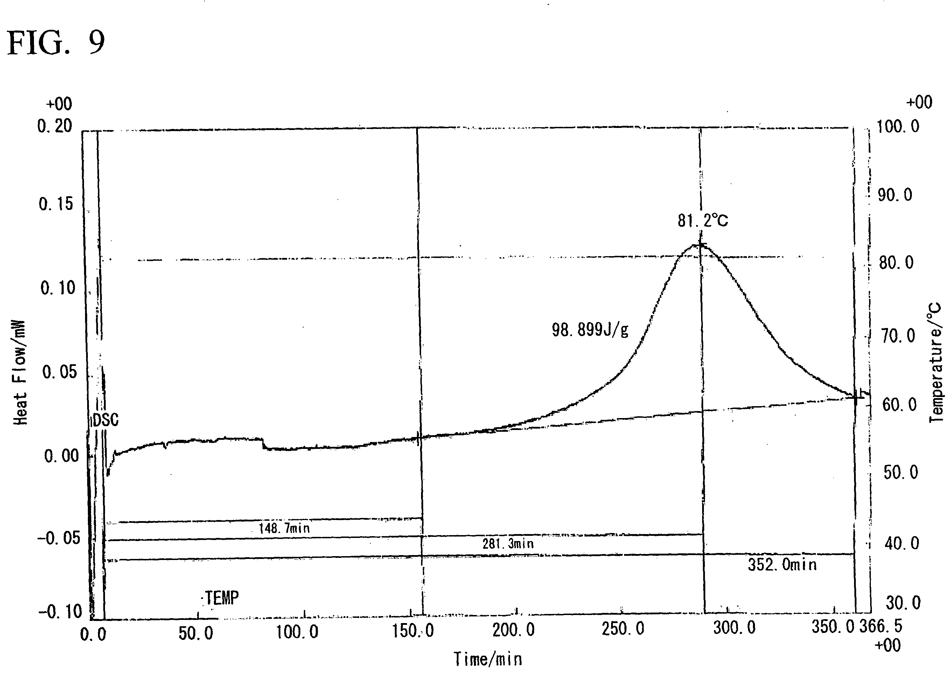

20 ml of a methanol solution containing 10 mmol (0.82 g) of 2-methylimidazole was added to 20 ml of a methanol solution containing 5 mmol (1.05 g) of 5-nitroisophthalic acid under conditions of heated reflux with stirring. Although heating is subsequently stopped, crystals precipitate almost immediately, the mixture was left to stand overnight at room temperature, and then filtered and dried under vacuum, yielding a clathrate (1.2 g, 64%). Analysis of the obtained clathrate by NMR revealed 1:1 clathrate crystals. A thermal analysis (TG/DTA) chart for the obtained clathrate crystals is shown in FIG. 7. Furthermore, a thermal analysis (DSC) chart upon temperature variation for the obtained clathrate crystals is shown in FIG. 8, whereas a thermal analysis (DSC) chart at a fixed temperature (80°C) is shown in FIG. 9.

### [Example 5]

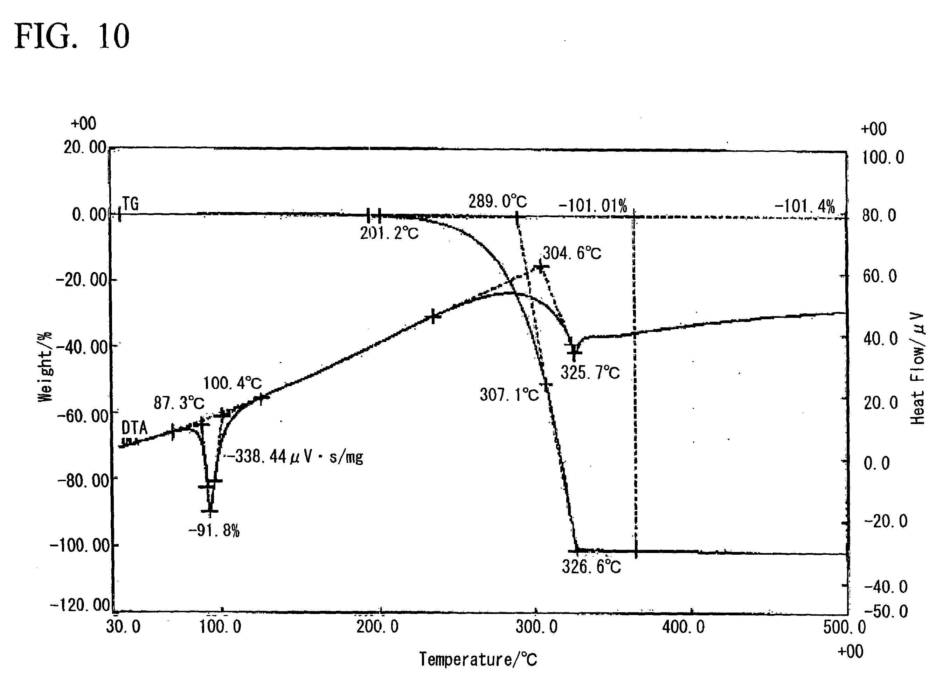

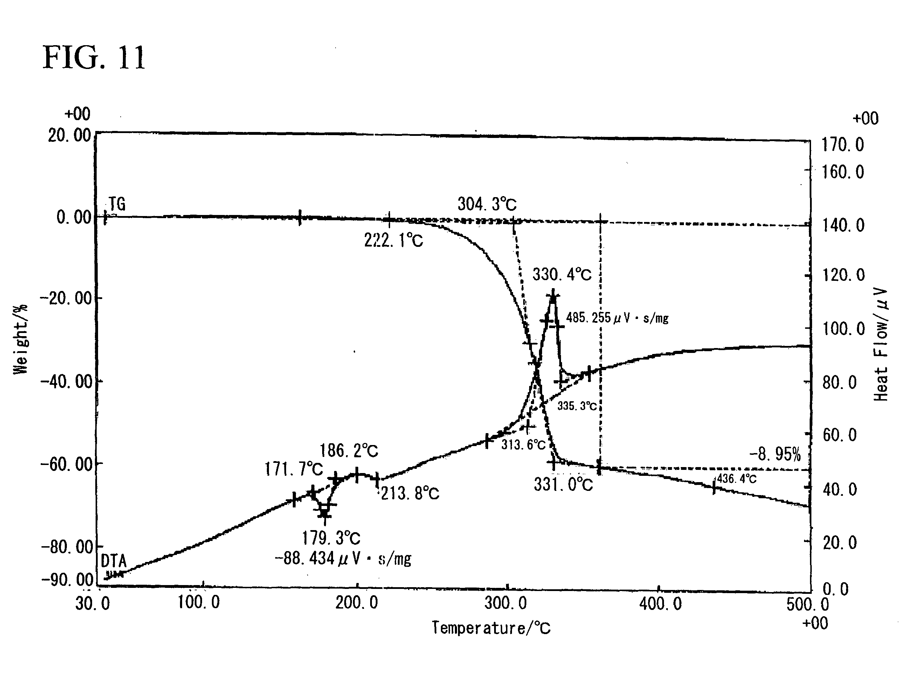

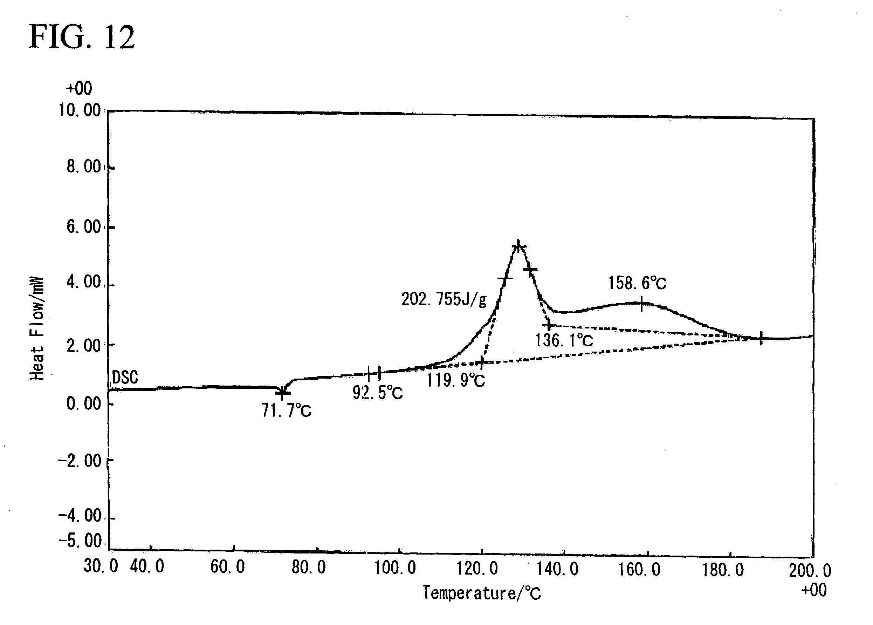

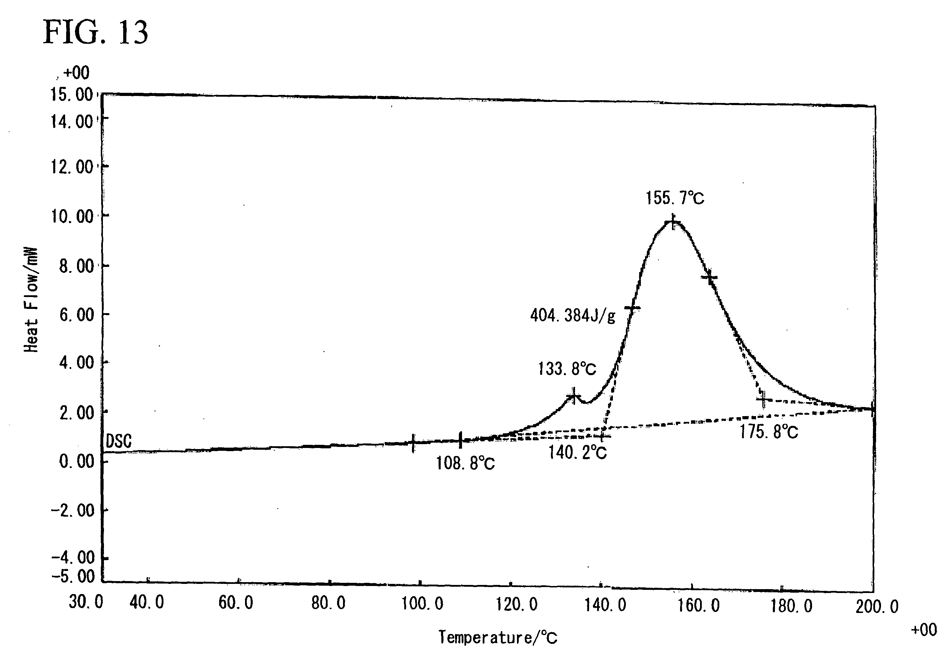

5 mmol (1.06 g) of 5-nitroisophthalic acid and 5 mmol (1.11 g) of 2-undecylimidazole were added to 40 ml of acetone, and the resulting mixture was stirred under heat and then left to stand overnight. After standing overnight, the mixture was filtered and dried under vacuum, yielding 1.98 g of a clathrate (1:1 clathrate).
A thermal analysis (TG/DTA) chart for only 2-undecylimidazole is shown in FIG. 10, whereas a thermal analysis (TG/DTA) chart for the obtained clathrate crystals is shown in FIG. 11. It is thought that because the melting point for 2-undecylimidazole was not observed in the chart of FIG. 11, the obtained crystals are a clathrate compound.
Furthermore, a thermal analysis (DSC) chart upon temperature variation for 2-undecylimidazole and an epoxy resin is shown in FIG. 12, whereas a thermal analysis (DSC) chart upon temperature variation for the obtained clathrate and an epoxy resin is shown in FIG. 13. The curing temperature in FIG. 13 was considerably higher than the curing temperature in FIG. 12, confirming that the clathrate structure generated an improvement in the one-pot stability.
The DSC charts were prepared by mixing 4% of the imidazole with a bisphenol A epoxy resin (YD-128), and then conducting measurements.

### [Example 6]

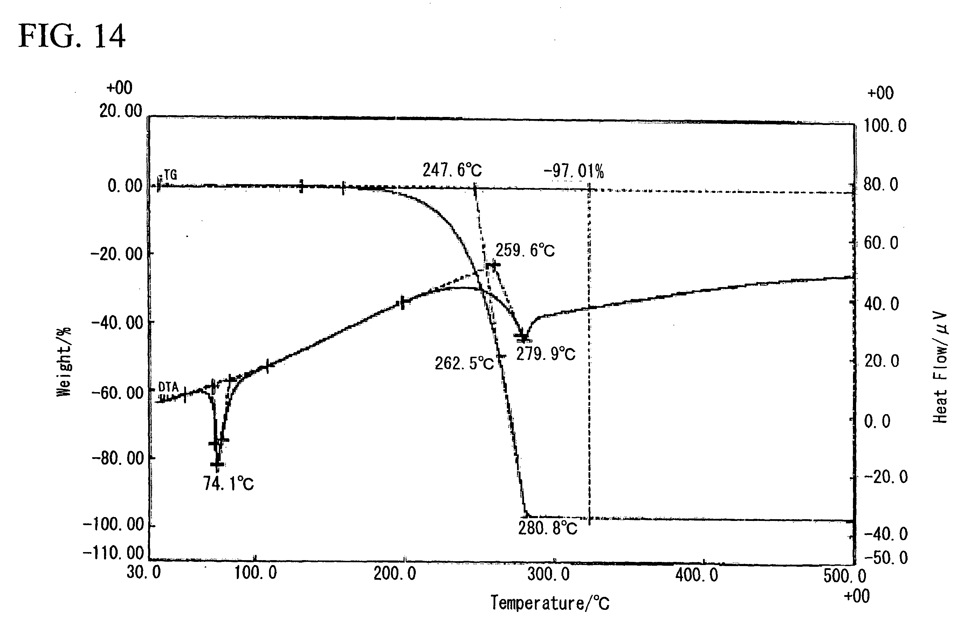

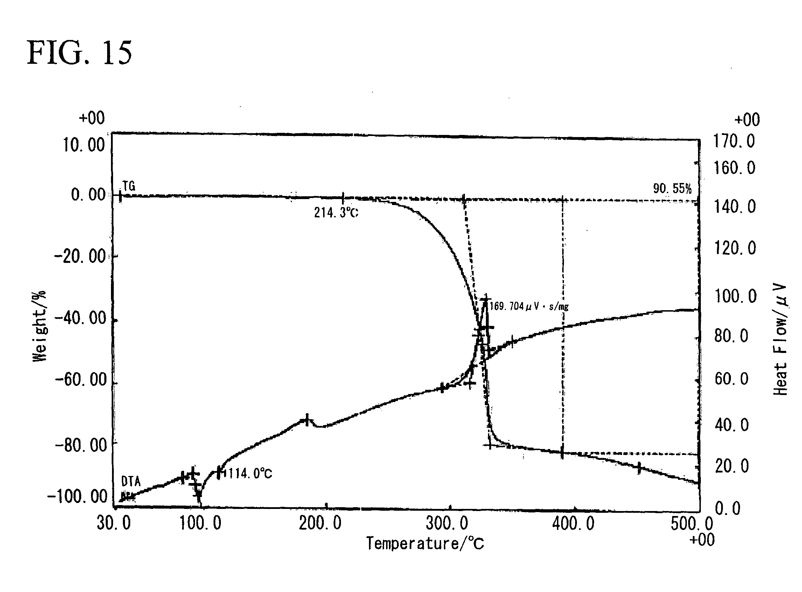

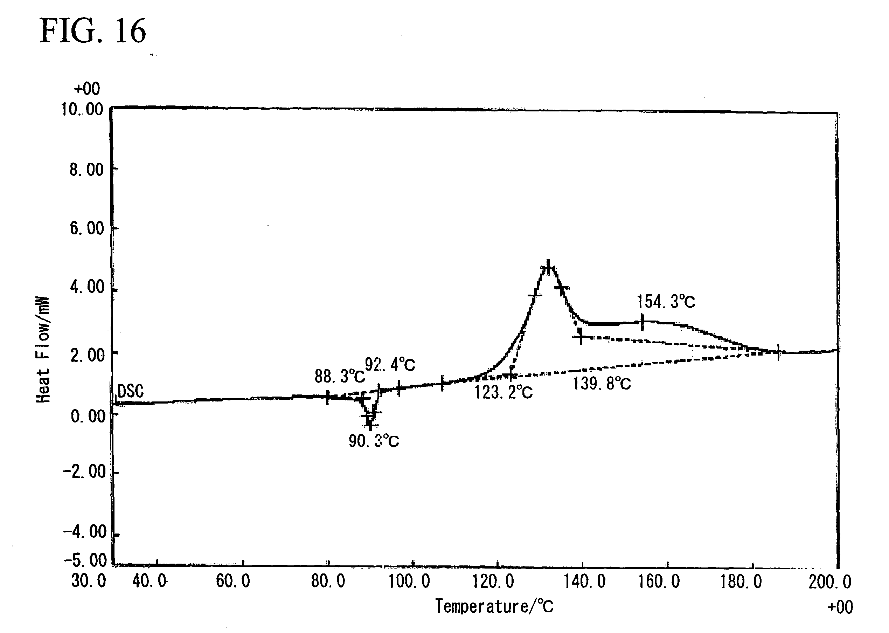

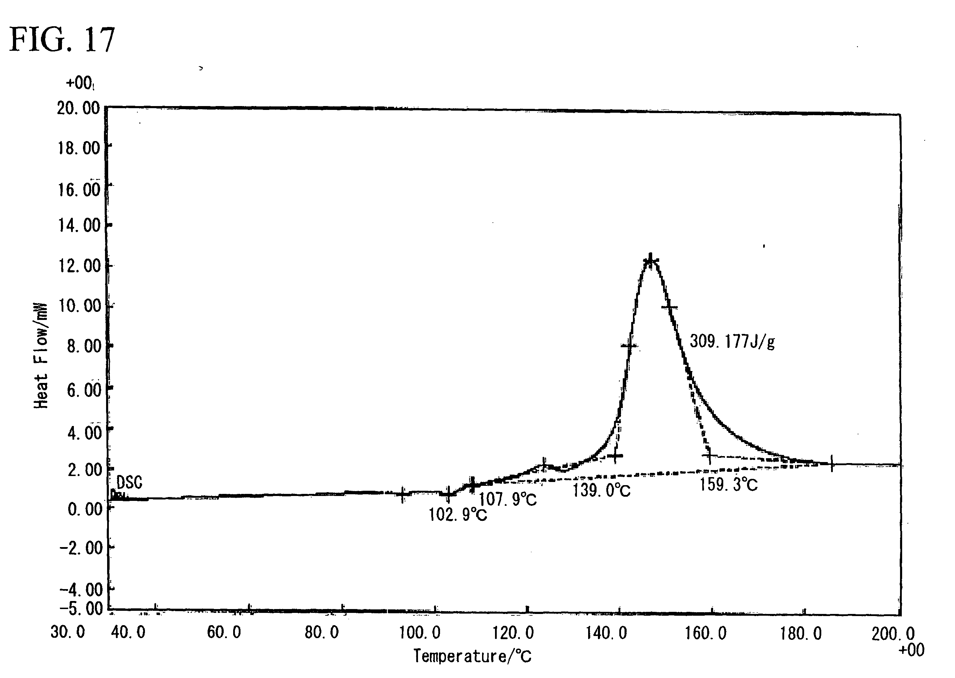

5 mmol (1.06 g) of 5-nitroisophthalic acid and 10 mmol (3.06 g) of 2-heptadecylimidazole were added to 30 ml of methanol, and the resulting mixture was stirred under heat and then left to stand overnight. After standing overnight, the mixture was filtered and dried under vacuum, yielding 3.16 g of a clathrate (1:2 clathrate).
A thermal analysis (TG/DTA) chart for only 2-heptadecylimidazole is shown in FIG. 14, whereas a thermal analysis (TG/DTA) chart for the obtained clathrate crystals is shown in FIG. 15. It is thought that because the melting point for 2-heptadecylimidazole was not observed in the chart of FIG. 15, the obtained crystals are a clathrate compound.
Furthermore, a thermal analysis (DSC) chart upon temperature variation for 2-heptadecylimidazole and an epoxy resin is shown in FIG. 16, whereas a thermal analysis (DSC) chart upon temperature variation for the obtained clathrate and an epoxy resin is shown in FIG. 17. The peaks in FIG. 16 and FIG. 17 are clearly different, confirming the difference in the structure obtained as a result of the clathrate structure.
The DSC charts were prepared by mixing 4% of the imidazole with a bisphenol A epoxy resin (YD-128), and then conducting measurements.

### [Comparative Example 1]

Using the same procedure as example 1, a thermal analysis (DSC) chart upon temperature variation and a thermal analysis (DSC) chart at a fixed temperature (80°C) were measured for 2-ethyl-4-methylimidazole (2E4MZ).

### [Comparative Example 2]

A methanol solution (200 ml) containing 125 mmol (49.8 g) of 1,1,2,2-tetrakis(4-hydroxyphenyl)ethane (TEP) was heated under reflux, and a methanol solution (20 ml) containing 267 mmol (29.4 g) of 2-ethyl-4-methylimidazole was then added dropwise to the refluxed solution. After stirring for one hour, the heating was stopped, and the mixture was left to stand overnight. Subsequently, the resulting mixture was filtered and dried under vacuum, yielding 54.6 g of a clathrate (TEP-2E4MZ). Using the same procedure as example 1, a thermal analysis (DSC) chart upon temperature variation and a thermal analysis (DSC) chart at a fixed temperature (80°C) were measured for the thus obtained clathrate.

### [Comparative Example 3]

15 mmol (2.5 g) of isophthalic acid and 16.5 mmol (1.8 g) of 2-ethyl-4-methylimidazole were dissolved in 15 ml of methanol under heating, and the resulting mixture was left to stand overnight. The precipitated crystals were then filtered and dried under vacuum, yielding 1.8 g of a clathrate (isophthalic acid-2E4MZ). Using the same procedure as example 1, a thermal analysis (DSC) chart upon temperature variation and a thermal analysis (DSC) chart at a fixed temperature (80°C) were measured for the thus obtained clathrate.

### [Comparative Example 4]

5 mmol (0.8 g) of terephthalic acid and 10 mmol (1.1 g) of 2-ethyl-4-methylimidazole were dissolved in 15 ml of methanol under heating, and the resulting mixture was left to stand overnight. The precipitated crystals were then filtered and dried under vacuum, yielding a clathrate (terephthalic acid-2E4MZ). Using the same procedure as example 1, a thermal analysis (DSC) chart upon temperature variation and a thermal analysis (DSC) chart at a fixed temperature (80°C) were measured for the thus obtained clathrate.

### [Comparative Example 5]

Using the same procedure as example 1, a thermal analysis (DSC) chart upon temperature variation and a thermal analysis (DSC) chart at a fixed temperature (80°C) were measured for 2-methylimidazole (2MZ).

### [Comparative Example 6]

75.0 g of 1,1,2,2-tetrakis(4-hydroxyphenyl)ethane (TEP), 31.0 g of 2-methylimidazole and 300 ml of ethyl acetate were mixed together, and the resulting mixture was heated under reflux for 3 hours. Subsequently, the mixture was left to stand overnight, and the resulting precipitate was then filtered and dried under vacuum, yielding 95 g of a clathrate (TEP-2MZ). Using the same procedure as example 1, a thermal analysis (DSC) chart upon temperature variation and a thermal analysis (DSC) chart at a fixed temperature (80°C) were measured for the thus obtained clathrate.

### [Comparative Example 7]

10 mmol (1.5 g) of 3,5-dihydroxybenzoic acid and 10 mmol (0.8 g) of 2-methylimidazole were dissolved in 50 ml of methanol under heating, and the resulting mixture was left to stand overnight. The precipitated crystals were then filtered and dried under vacuum, yielding a clathrate (3,5-dihydroxybenzoic acid-2MZ). Using the same procedure as example 1, a thermal analysis (DSC) chart upon temperature variation and a thermal analysis (DSC) chart at a fixed temperature (80°C) were measured for the thus obtained clathrate.

### [Comparative Example 8]

15 mmol (2.5 g) of isophthalic acid and 16.5 mmol (1.4 g) of 2-methylimidazole were added to 20 ml of methanol, and the resulting mixture was stirred under heat and then left to stand overnight. The resulting precipitate was then filtered and dried under vacuum, yielding 2.8 g of a clathrate (isophthalic acid-2MZ). Using the same procedure as example 1, a thermal analysis (DSC) chart upon temperature variation and a thermal analysis (DSC) chart at a fixed temperature (80°C) were measured for the thus obtained clathrate.

Table 1 below shows, in graphic form, the values for the reaction start temperature, the peak top, and the reaction end temperature read from the charts shown in FIG. 2 (example 1), FIG. 5 (example 2) and FIG. 8 (example 4), as well as the same values for the comparative examples also shown in graphic form.

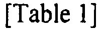

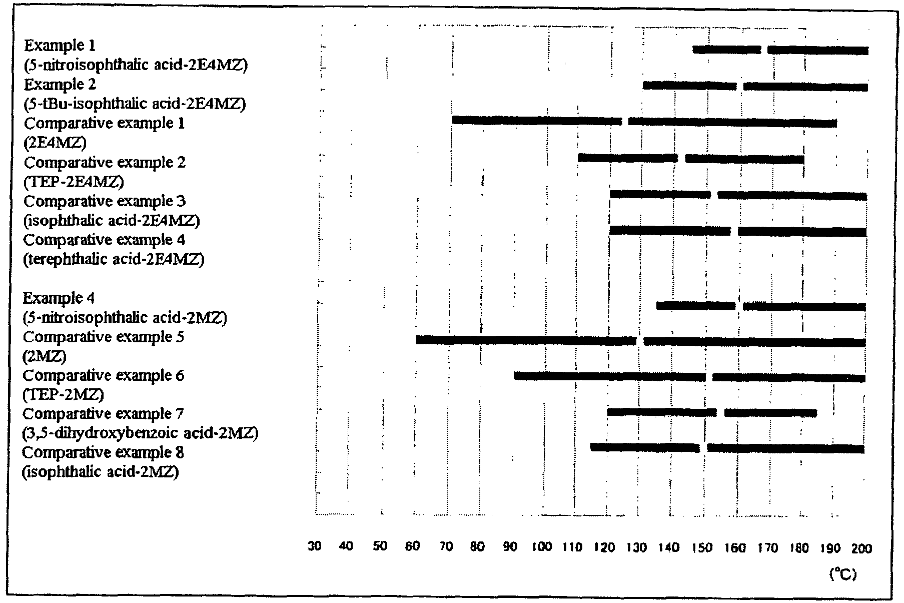

From the figures and Table 1, it is evident that the clathrates according to the examples exhibit a higher reaction start temperature, which indicates an improvement in the one-pot stability. Furthermore, the clathrates according to the examples also have a small temperature difference between the reaction start temperature and the peak top, and it is thought that this indicates a higher degree of reactivity for the epoxy rings.

Table 2 below shows, in graphic form, the values for the reaction start temperature, the peak top, and the reaction end temperature read from the charts shown in FIG. 3 (example 1), FIG. 6 (example 2) and FIG. 9 (example 4), as well as the same values for the comparative examples also shown in graphic form.

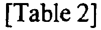

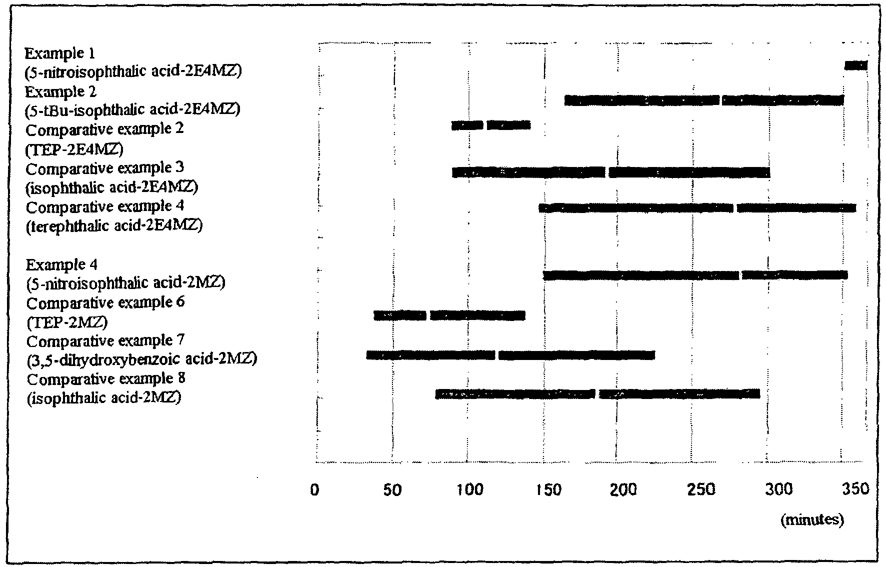

The fixed temperature of 80°C is a typical temperature used during mixing of an epoxy resin and a clathrate, and therefore suppressing reaction at this temperature is extremely important. From the figures and Table 2, it is evident that the clathrates according to the examples exhibit much longer time before the reaction starts and before the reaction peak, indicating an extremely favorable level of one-pot stability.

## Claims

1. A clathrate compound comprising at least an isophthalic acid compound represented by a formula (I) shown below: 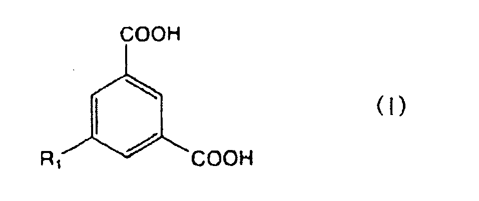 wherein R₁ represents a C1 to C6 alkyl group, C1 to C6 alkoxy group, nitro group or hydroxyl group, and
an imidazole compound represented by a formula (II) shown below: 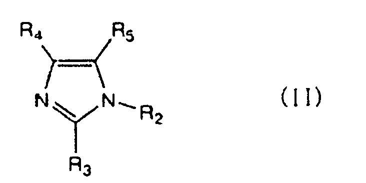 wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent
provided that the clathrate compound is neither [Co(5-nbdch(imH)₂]ₙ nor [Cu₃(5-nbdc)₂(OH)₂(imH)₂]ₙ, wherein 5-nbdcH₂ represents 5-nitro-1,3-benzenedicarboxylic acid and imH represents imidazole.

2. The compound according to claim 1, wherein said isophthalic acid compound represented by said formula (I) is 5-t-butylisophthalic acid or 5-nitroisophthalic acid.

3. The compound according to claim 1 or 2, wherein said imidazole compound represented by said formula (II) is 2-ethyl-4-methylimidazole, 2-methylimidazole, 1-benzyl-2-methylimidazole, 2-heptadecylimidazole, 2-undecylimidazole or 2-phenyl-4-methyl-5-hydroxymethylimidazole.

4. The compound according to any one of claims 1 to 3, wherein R₂ in said formula (II) is a hydrogen atom.

5. The compound according to any one of claims 1 to 4, wherein said compound is in a powdered form.

6. A curing catalyst for an epoxy resin, comprising a clathrate compound according to any one of claims 1 to 5.

7. A composition for forming a cured epoxy resin comprising:
(A) an epoxy resin, and
(B) a clathrate compound comprising at least an isophthalic acid compound represented by a formula (I) shown below: 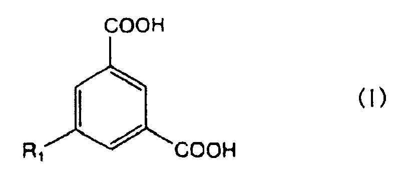 wherein R₁ represents a C1 to C6 alkyl group, C1 to C6 alkoxy group, nitro group or hydroxyl group, and
an imidazole compound represented by a formula (II) shown below: 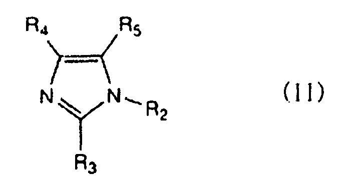 wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent.

8. The composition for forming a cured epoxy resin according to claim 7, wherein an amount of said imidazole compound represented by said formula (II) within component (B) is within a range from 0.01 to 1.0 mol relative to 1 mol of epoxy rings within said epoxy resin of component (A).

9. The composition for forming a cured epoxy resin according to claim 7 or 8, wherein said isophthalic acid compound represented by said formula (I) is 5-t-butylisophthalic acid or 5-nitroisophthalic acid.

10. The composition for forming a cured epoxy resin according to any one of claims 7 to 9, wherein said imidazole compound represented by said formula (II) is 2-ethyl-4-methylimidazole, 2-methylimidazole, 1-benzyl-2-methylimidazole, 2-heptadecylimidazole, 2-undecylimidazole or 2-phenyl-4-methyl-5-hydroxymethylimidazole.

11. A method of producing a cured epoxy resin, comprising curing a composition for forming a cured epoxy resin according to any one of claims 7 to 10 by conducting a heat treatment.

12. The method of producing a cured epoxy resin according to claim 11, wherein a heating temperature during said heat treatment is within a range from 60 to 250°C.

13. A cured epoxy resin, obtained using a method according to claim 11 or 12.

14. A compound that can be obtained after dissolving or suspending at least an isophthalic acid compound represented by a formula (I) shown below: 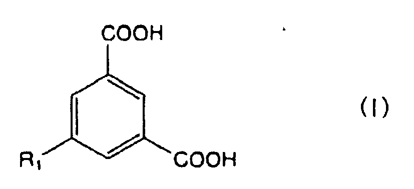 wherein R₁ represents a C1 to C6 alkyl group, C1 to C6 alkoxy group, nitro group or hydroxyl group, and
an imidazole compound represented by a formula (II) shown below: 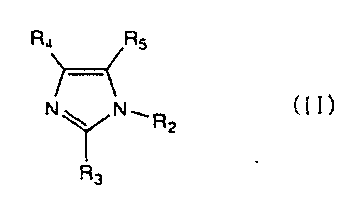 wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent
in a solvent and subsequently conducting heating
provided that the clathrate compound is neither [Co(5-nbdc)₂(imH)₂]ₙ nor [Cu₃(5-nbdC)₂(OH)₂(imH)₂]ₙ, wherein 5-nbdcH₂ represents 5-nitro-1,3-benzenedicarboxylic acid and imH represents imidazole.

15. The compound according to claim 14, which can be obtained by dissolving or suspending said isophthalic acid compound represented by said formula (I) and said imidazole compound represented by said formula (II) in a solvent, conducting heating, and then performing a crystallization.

16. Use of a host compound for manufacturing a clathrate compound, **characterized in that** the host compound is represented by a formula (I) shown below: 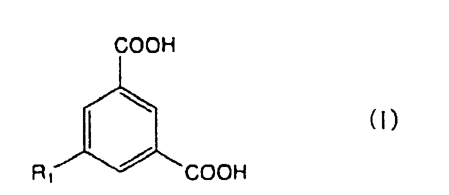 wherein R₁ represents a nitro group or a C4 alkyl group
provided that the clathrate compound is neither [Co(5-nbdc)₂(imH)₂]ₙ nor [Cu₃(5-nbdc)₂(OH)₂(imH)₂]ₙ, wherein 5-nbdcH₂ represents 5-nitro-1,3-benzenedicarboxylic acid and imH represents imidazole.

17. The use according to claim 16, wherein said C4 alkyl group is a t-butyl group.

18. Use of a clathrate compound comprising at least an isophthalic acid compound represented by a formula (I) shown below: 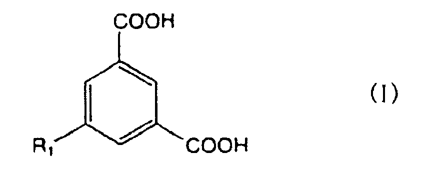 wherein R₁ represents a C1 to C6 alkyl group, C1 to C6 alkoxy group, nitro group or hydroxyl group, and
an imidazole compound represented by a formula (II) shown below: 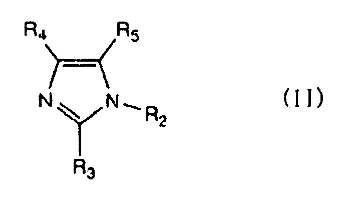 wherein R₂ represents a hydrogen atom, C1 to C10 alkyl group, phenyl group, benzyl group or cyanoethyl group, and R₃ to R₅ each independently represents a hydrogen atom, nitro group, halogen atom, or a C1 to C20 alkyl group, phenyl group, benzyl group or C1 to C20 acyl group that may have a substituent, as curing catalyst.

## Patentansprüche

1. Clathrat-Verbindung, umfassend zumindest eine Isophthalsäureverbindung, dargestellt durch eine unten gezeigte Formel (I): 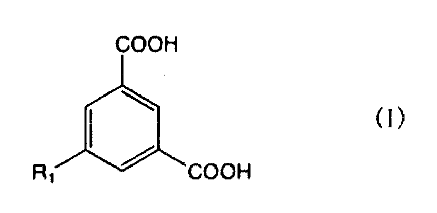 worin R₁ eine C1 bis C6 Alkylgruppe, C1 bis C6 Alkoxygruppe, Nitrogruppe oder Hydroxylgruppe darstellt, und
eine Imidazolverbindung, dargestellt durch eine unten gezeigte Formel (II): 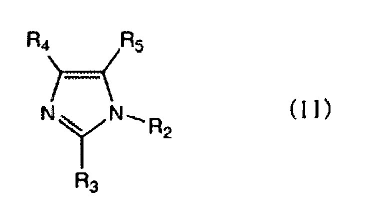 worin R₂ ein Wasserstoffatom, C1 bis C10 Alkylgruppe, Phenylgruppe, Benzylgruppe oder Cyanoethylgruppe darstellt, und R₃ bis R₅ jeweils unabhängig ein Wasserstoffatom, Nitrogruppe, Halogenatom, oder eine C1 bis C20 Alkylgruppe, Phenylgruppe, Benzylgruppe oder C1 bis C20 Acylgruppe, die einen Substituenten haben kann, darstellt,
vorausgesetzt, dass die Clathrat-Verbindung weder [Co(5-nbdc)₂(imH)₂]ₙ noch [Cu₃(5-nbdc)₂(OH)₂(imH)₂]ₙ ist, wobei 5-nbdcH₂ 5-Nitro-1,3-benzoldicarbonsäure und imH Imidazol darstellt.

2. Die Verbindung nach Anspruch 1, wobei diese Isophthalsäureverbindung, die durch diese Formel (I) dargestellt ist, 5-t-Butylisophthalsäure oder 5-Nitroisophthalsäure ist.

3. Die Verbindung nach Anspruch 1 oder 2, wobei diese Imidazolverbindung, dargestellt durch diese Formel (II), 2-Ethyl-4-methylimidazol, 2-Methylimidazol, 1-Benzyl-2-methylimidazol, 2-Heptadecylimidazol, 2-Undecylimidazol oder 2-Phenyl-4-methyl-5-hydroxymethylimidazol ist.

4. Die Verbindung nach einem der Ansprüche 1 bis 3, wobei R₂ in dieser Formel (II) ein Wasserstoffatom ist.

5. Die Verbindung nach einem der Ansprüche 1 bis 4, wobei diese Verbindung in einer puiverisierten Form ist.

6. Härtungskatalysator für ein Epoxidharz, umfassend eine Clathratverbindung gemäß einem der Ansprüche 1 bis 5.

7. Zusammensetzung zur Bildung eines gehärteten Epoxidharzes, umfassend:
(A) ein Epoxidharz, und
(B) eine Clathrat-Verbindung, umfassend zumindest eine Isophthalsäureverbindung, dargestellt durch eine unten gezeigte Formel (I): 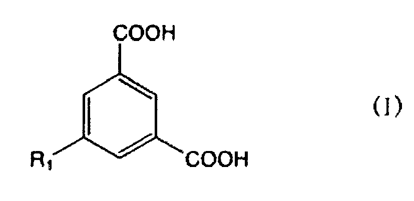 worin R₁ eine C1 bis C6 Alkylgruppe, C1 bis C6 Alkoxygruppe, Nitrogruppe oder Hydroxylgruppe darstellt, und
eine Imidazolverbindung, dargestellt durch eine unten gezeigte Formel (II): 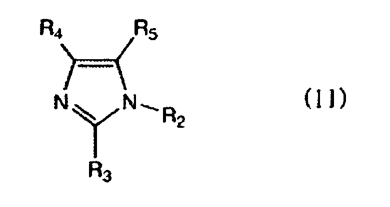 worin R₂ ein Wasserstoffatom, C1 bis C10 Alkylgruppe, Phenylgruppe, Benzylgruppe oder Cyanoethylgruppe darstellt, und R₃ bis R₅ jeweils unabhängig ein Wasserstoffatom, Nitrogruppe, Halogenatom, oder eine C1 bis C20 Alkylgruppe, Phenylgruppe, Benzylgruppe oder C1 bis C20 Acylgruppe, die einen Substituenten haben kann, darstellt.

8. Die Zusammensetzung zur Bildung eines gehärteten Epoxidharzes nach Anspruch 7, wobei eine Menge dieser Imidazolverbindung, dargestellt durch diese Formel (II), innerhalb der Komponente (B) in einem Bereich von 0,01 bis 1,0 Mol bezogen auf 1 Mol an Epoxidringen innerhalb dieses Epoxidharzes von Komponente (A) ist.

9. Die Zusammensetzung zur Bildung eines gehärteten Epoxidharzes nach Anspruch 7 oder 8, wobei diese Isophthalsäureverbindung, die durch diese Formel (I) dargestellt ist, 5-t-Butylisophthalsäure oder 5-Nitroisophthalsäure ist.

10. Die Zusammensetzung zur Bildung eines gehärteten Epoxidharzes nach einem der Ansprüche 7 bis 9, wobei diese Imidazolverbindung, dargestellt durch diese Formel (II), 2-Ethyl-4-methylimidazol, 2-Methylimidazol, 1-Benzyl-2-methylimidazol, 2-Heptadecylimidazol, 2-Undecylimidazol oder 2-Phenyl-4-methyl-5-hydroxymethylimidazol ist.

11. Verfahren zur Herstellung eines gehärteten Epoxidharzes, umfassend das Härten einer Zusammensetzung zur Bildung eines gehärteten Epoxidharzes gemäß einem der Ansprüche 7 bis 10 unter Durchführen einer Wärmebehandlung.

12. Das Verfahren zur Herstellung eines gehärteten Epoxidharzes nach Anspruch 11, wobei eine Erwärmungstemperatur während dieser Wärmebehandlung innerhalb eines Bereichs von 60 bis 250°C liegt.

13. Gehärtetes Epoxidharz, erhalten unter Verwendung eines Verfahrens gemäß Anspruch 11 oder 12.

14. Verbindung, die erhalten werden kann nach Auflösen oder Suspendieren von zumindest einer Isophthalsäureverbindung, dargestellt durch eine unten gezeigte Formel (I): 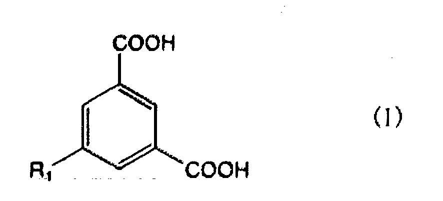 worin R₁ eine C1 bis C6 Alkylgruppe, C1 bis C6 Alkoxygruppe, Nitrogruppe oder Hydroxylgruppe darstellt, und
eine Imidazolverbindung, dargestellt durch eine unten gezeigte Formel (II): 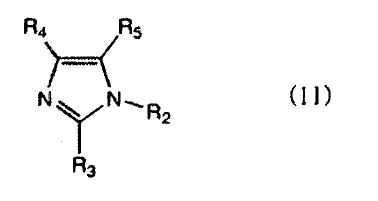 worin R₂ ein Wasserstoffatom, C1 bis C10 Alkylgruppe, Phenylgruppe, Benzylgruppe oder Cyanoethylgruppe darstellt, und R₃ bis R₅ jeweils unabhängig ein Wasserstoffatom, Nitrogruppe, Halogenatom, oder eine C1 bis C20 Alkylgruppe, Phenylgruppe, Benzylgruppe oder C1 bis C20 Acylgruppe, die einen Substituenten haben kann, darstellt,
in einem Lösungsmittel und nachfolgender Durchführung einer Erwärmung, vorausgesetzt, dass die Clathrat-Verbindung weder [Co(5-nbdC)₂(imH)₂]ₙ noch [Cu₃(5-nbdc)₂(OH)₂(imH)₂]ₙ ist, wobei 5-nbdcH₂ 5-Nitro-1,3-benzoldicarbonsäure und imH Imidazol darstellt.

15. Die Verbindung nach Anspruch 14, welche erhalten werden kann durch Auflösen oder Suspendieren dieser Isophthalsäureverbindung, dargestellt durch diese Formel (I), und dieser Imidazolverbindung, dargestellt durch diese Formel (II), in einem Lösungsmittel, Durchführen von Erwärmung und dann Ausführen einer Kristallisation.

16. Verwendung einer Wirtsverbindung zur Herstellung einer Clathrat-Verbindung, dadurch charakterisiert, dass die Wirtsverbindung durch eine unten gezeigte Formel (I) dargestellt ist: 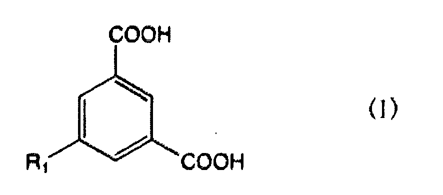 worin R₁ eine Nitrogruppe oder eine C4 Alkylgruppe darstellt,
vorausgesetzt, dass die Clathrat-Verbindung weder [Co(5-nbdc)₂(imH)₂]ₙ noch [Cu₃(5-nbdc)₂(OH)₂(imH)₂]ₙ ist, wobei 5-nbdcH₂ 5-Nitro-1,3-benzoldicarbonsäure und imH Imidazol darstellt.

17. Die Verwendung nach Anspruch 16, wobei diese C4 Alkylgruppe eine t-Butylgruppe ist.

18. Verwendung einer Clathrat-Verbindung, umfassend zumindest eine Isophthalsäureverbindung, dargestellt durch eine unten gezeigte Formel (I): 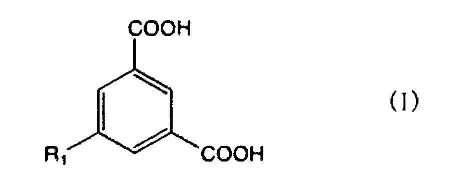 worin R₁ eine C1 bis C6 Alkylgruppe, C1 bis C6 Alkoxygruppe, Nitrogruppe oder Hydroxylgruppe darstellt, und
eine Imidazolverbindung, dargestellt durch eine unten gezeigte Formel (II): 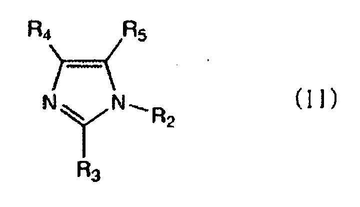 worin R₂ ein Wasserstoffatom, C1 bis C10 Alkylgruppe, Phenylgruppe, Benzylgruppe oder Cyanoethylgruppe darstellt, und R₃ bis R₅ jeweils unabhängig ein Wasserstoffatom, Nitrogruppe, Halogenatom, oder eine C1 bis C20 Alkylgruppe, Phenylgruppe, Benzylgruppe oder C1 bis C20 Acylgruppe, die einen Substituenten haben kann, darstellt, als Härtungskatalysator.

## Revendications

1. Composé clathrate comprenant au moins un composé d'acide isophtalique représenté par une formule (I) montrée ci-dessous : 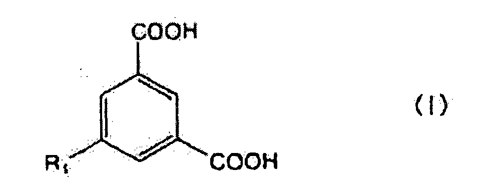 dans laquelle R₁ représente un groupe alkyle en C1-C6, un groupe alcoxy en C1-C6, un groupe nitro ou un groupe hydroxyle, et
un composé d'imidazole représenté par une formule (II) montrée ci-dessous : 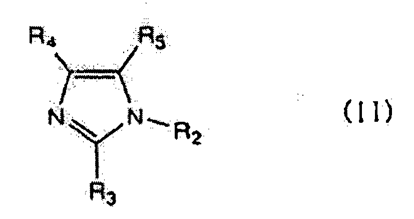 dans laquelle R₂ représente un atome d'hydrogène, un groupe alkyle en C1-C10, un groupe phényle, un groupe benzyle ou un groupe cyanoéthyle, et R₃ à R₅ représentent chacun indépendamment un atome d'hydrogène, un groupe nitro, un atome d'halogène, ou un groupe alkyle en C1-C20, un groupe phényle, un groupe benzyle ou un groupe acyle en C1-C20 qui peut avoir un substituant,
sous réserve que le composé clathrate n'est ni [CO(5-nbdc)₂(imH)₂]ₙ, ni [Cu₃(5-nbdc)₂(OH)₂(imH)₂]ₙ, dans lesquels 5-nbdcH₂ représente un acide 5-nitro-1,3-benzènedicarboxylique et imH représente un imidazole.

2. Composé selon la revendication 1, dans lequel ledit composé d'acide isophtalique représenté par ladite formule (I) est l'acide 5-t-butylisophtalique ou l'acide 5-nitroisophtalique.

3. Composé selon la revendication 1 ou 2, dans lequel ledit composé d'imidazole représenté par ladite formule (II) est le 2-éthyl-4-méthylimidazole, le 2-méthylimidazole, le 1-benzyl-2-méthylimidazole, le 2-heptadécylimidazole, le 2-undécylimidazole ou le 2-phényl-4-méthyl-5-hydroxyméthylimidazole.

4. Composé selon l'une quelconque des revendications 1 à 3, dans lequel R₂ dans ladite formule (II) est un atome d'hydrogène.

5. Composé selon l'une quelconque des revendications 1 à 4, dans lequel ledit composé est sous une forme pulvérulente.

6. Catalyseur de durcissement pour une résine époxy, comprenant un composé clathrate selon l'une quelconque des revendications 1 à 5.

7. Composition pour former une résine époxy durcie comprenant :
(A) une résine époxy, et
(B) un composé clathrate comprenant au moins un composé d'acide isophtalique représenté par une formule (I) montrée ci-dessous : 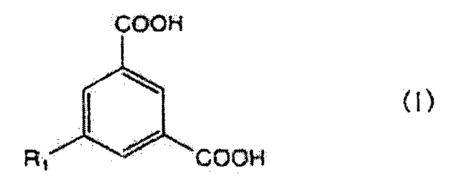 dans laquelle R₁ représente un groupe alkyle en C1-C6, un groupe alcoxy en C1-C6, un groupe nitro ou un groupe hydroxyle, et
un composé d'imidazole représenté par une formule (II) montrée ci-dessous : 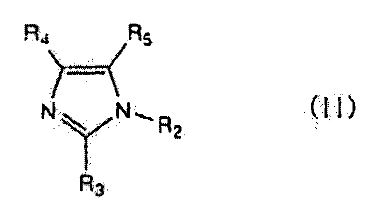 dans laquelle R₂ représente un atome d'hydrogène, un groupe alkyle en C1-C10, un groupe phényle, un groupe benzyle ou un groupe cyanoéthyle, et R₃ à R₅ représentent chacun indépendamment un atome d'hydrogène, un groupe nitro, un atome d'halogène, ou un groupe alkyle en C1-C20, un groupe phényle, un groupe benzyle ou un groupe acyle en C1-C20 qui peut avoir un substituant.

8. Composition pour former une résine époxy durcie selon la revendication 7, dans laquelle une quantité dudit composé d'imidazole représenté par ladite formule (II) au sein du composant (B) est dans une plage de 0,01 à 1,0 mole pour 1 mole de noyaux époxy au sein de ladite résine époxy du composant (A).

9. Composition pour former une résine époxy durcie selon la revendication 7 ou 8, dans laquelle ledit composé d'acide isophtalique représenté par ladite formule (I) est l'acide 5-t-butylisophtalique ou l'acide 5-nitroisophtalique.

10. Composition pour former une résine époxy durcie selon l'une quelconque des revendications 7 à 9, dans laquelle ledit composé d'imidazole représenté par ladite formule (II) est le 2-éthyl-4-méthylimidazole, le 2-méthylimidazole, le 1-benzyl-2-méthylimidazole, le 2-heptadécylimidazole, le 2-undécylimidazole ou le 2-phényl-4-méthyl-5-hydroxyméthylimidazole.

11. Procédé de production d'une résine époxy durcie, comprenant le durcissement d'une composition pour former une résine époxy durcie selon l'une quelconque des revendications 7 à 10 en exécutant un traitement thermique.

12. Procédé de production d'une résine époxy durcie selon la revendication 11, dans lequel une température de chauffage pendant ledit traitement thermique est dans une plage de 60 à 250 °C.

13. Résine époxy durcie, obtenue en utilisant un procédé selon la revendication 11 ou 12.

14. Composé qui peut être obtenu après dissolution ou mise en suspension d'au moins un composé d'acide isophtalique représenté par une formule (I) montrée ci-dessous : 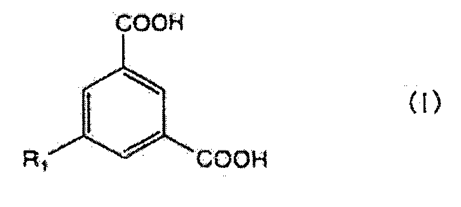 dans laquelle R₁ représente un groupe alkyle en C1-C6, un groupe alcoxy en C1-C6, un groupe nitro ou un groupe hydroxyle, et
un composé d'imidazole représenté par une formule (II) montrée ci-dessous : 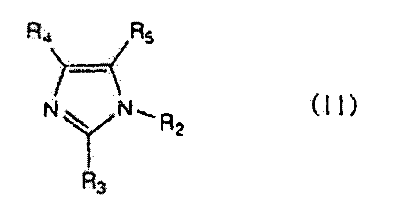 dans laquelle R₂ représente un atome d'hydrogène, un groupe alkyle en C1-C10, un groupe phényle, un groupe benzyle ou un groupe cyanoéthyle, et R₃ à R₅ représentent chacun indépendamment un atome d'hydrogène, un groupe nitro, un atome d'halogène, ou un groupe alkyle en C1-C20, un groupe phényle, un groupe benzyle ou un groupe acyle en C1-C20 qui peut avoir un substituant
dans un solvant et ensuite exécution d'un chauffage,
sous réserve que le composé clathrate n'est ni [CO(5-nbdc)₂(imH)₂]ₙ, ni [Cu₃(5-nbdc)₂(OH)₂(imH)₂]ₙ, dans lesquels 5-nbdcH₂ représente un acide 5-nitro-1,3-benzènedicarboxylique et imH représente un imidazole.

15. Composé selon la revendication 14, qui peut être obtenu par dissolution ou mise en suspension dudit composé d'acide isophtalique représenté par ladite formule (I) et dudit composé d'imidazole représenté par ladite formule (II) dans un solvant, exécution d'un chauffage, et ensuite exécution d'une cristallisation.

16. Utilisation d'un composé hôte pour fabriquer un composé clathrate, **caractérisé en ce que** le composé hôte est représenté par une formule (I) montrée ci-dessous : 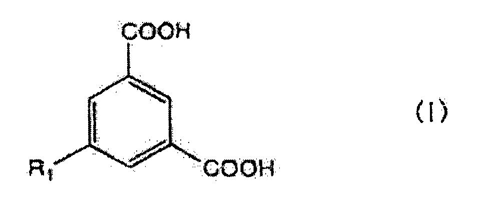 dans laquelle R₁ représente un groupe nitro ou un groupe alkyle en C4,
sous réserve que le composé clathrate n'est ni [CO(5-nbdc)₂(imH)₂]ₙ, ni [Cu₃(5-nbdc)₂(OH)₂(imH)₂]ₙ, dans lesquels 5-nbdcH₂ représente un acide 5-nitro-1,3-benzènedicarboxylique et imH représente un imidazole.

17. Utilisation selon la revendication 16, dans laquelle ledit groupe alkyle en C4 est un groupe t-butyle.

18. Utilisation d'un composé clathrate comprenant au moins un composé d'acide isophtalique représenté par une formule (I) montrée ci-dessous : 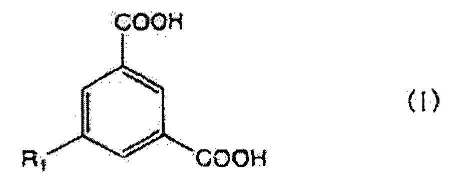 dans laquelle R₁ représente un groupe alkyle en C1-C6, un groupe alcoxy en C1-C6, un groupe nitro ou un groupe hydroxyle, et
un composé d'imidazole représenté par une formule (II) montrée ci-dessous : 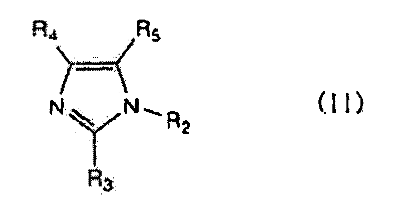 dans laquelle R₂ représente un atome d'hydrogène, un groupe alkyle en C1-C10, un groupe phényle, un groupe benzyle ou un groupe cyanoéthyle, et R₃ à R₅ représentent chacun indépendamment un atome d'hydrogène, un groupe nitro, un atome d'halogène, ou un groupe alkyle en C1-C20, un groupe phényle, un groupe benzyle ou un groupe acyle en C1-C20 qui peut avoir un substituant, comme un catalyseur de durcissement.
